# EUROPEAN PATENT SPECIFICATION

(11) **EP 0 808 317 B1**
(45) Date of publication and mention of the grant of the patent: **10.09.2003**
(21) Application number: 96901958.7
(22) Date of filing: 07.02.1996
(51) Int. Cl.: C07D 495/04, A61K 31/505

(54) **THIENOPYRIMIDINE DERIVATIVES, THEIR PRODUCTION AND USE**
THIENOPYRIMIDINDERIVATE, IHRE HERSTELLUNG UND VERWENDUNG
DERIVES A BASE DE THIENOPYRIMIDINE, LEUR PRODUCTION ET LEURS UTILISATIONS

(30) Priority: 08.02.1995 JP 2071795; 28.02.1995 JP 4015195; 14.04.1995 WO PCT/JP95/00728; 19.10.1995 JP 27163895
(43) Date of publication of application: 26.11.1997
(73) Proprietor: Takeda Chemical Industries, Ltd., Osaka-shi, Osaka 541-0045 (JP)
(72) Inventor: FURUYA, Shuichi, Ibaraki 305 (JP); CHOH, Nobuo, Ibaraki 305 (JP); KATO, Koichi, Ibaraki 305 (JP); HINUMA, Shuji, Ibaraki 305 (JP)
(74) Representative: von Kreisler, Alek, Dipl.-Chem.
(86) International application number: JP9600263
(87) International publication number: WO96024597

(56) References cited:
- EP-A- 0 443 568
- EP-A- 0 640 606
- DE-A- 2 006 505

## Description

### Technical Field

The present invention relates to novel thienopyrimidine derivatives and salts thereof. The present invention further relates to methods for manufacturing the thienopyrimidine derivatives and the salts thereof, and pharmaceutical compositions containing the thienopyrimidine derivatives.

### Background Art

Secretion of anterior pituitary hormone is controlled by peripheral hormones secreted from target organs for the respective hormones and by secretion-accelerating or -inhibiting hormone from the hypothalamus, which is the upper central organ of the anterior lobe of the pituitary (in this specification, these hormones are collectively called "hypothalamic hormone"). At the present stage, as hypothalamic hormones, nine kinds of hormones including, for example, thyrotropin releasing hormone (TRH) or gonadotropin releasing hormone {GnRH: sometimes called LH-RH (luteinizing hormone releasing hormone)} have been confirmed (cf. Seirigaku 2, compiled by M. Iriku and K Toyama, published by Bunkohdo, p610-618, 1986). These hypothalamic hormones are assumed to show their actions via the receptor which is considered to exist in the anterior lobe of the pituitary (cf. ibid), and studies of receptor genes specific to these hormones, including those of humans, have been developed (Receptor Kiso To Rinshô, compiled by H. Imura, et al., published by Asakura Shoten, p297-304, 1993). Accordingly, antagonists or agonists specifically and selectively acting on these receptors control the action of hypothalamic hormone and the secretion of anterior pituitary hormone. As a result, they are expected to be useful as prophylactic and therapeutic agents of anterior pituitary hormone dependent diseases.

Leuprorelin acetate (Fujino et al., Biological and Biophysical Research Communications, Vol.60, 00.406-413, 1974; Oliver, R.T.D. et al., British Journal of Cancers, Vol.59, p.823, 1989; and Toguchi et al., Journal of International Medical Research, Vol.18, pp.35-41), which is a highly potent derivative of gonadotropic hormone-releasing hormone, one of the hypothalamic hormones, (hereinafter sometimes abbreviated as GnRH) (Schally A. V. et at., Journal of Biological Chemistry, Vol. 246, pp.7230-7236, 1971; and Burgus, R. et al., Proceeding of Natural Academic Science, USA, Vol.69, pp278-282, 1972), by administration of multiple doses, lowers release of gonadotropic hormone in the pituitary, causing a lowering of reactivity of gonadotropic hormone in the sperm and ovary tissue to suppress secretion of testosterone and estrogen. Leuprorelin acetate has, therefore, been known to show antitumor activity on such hormone-dependent cancers as prostate cancer, and has been widely used in the clinical field. Leuprorelin acetate has been widely used clinically also as a therapeutic agent of e.g. endometriosis and precocious puberty. The high antitumor activity of leuprorelin acetate is assumed to be due to its high resistance, as compared with natural GnRH, against protease,and to its high affinity for GnRH receptor causing desensitization of GnRH due to decrease in number of receptors. However, as leuprorelin acetate is an ultra-agonist of GnRH receptors, it has been known that, immediately after the first administration, a transient aggravation accompanied with a rise of serum testosterone concentration due to pituitary-gonadotropic action (acute action) is observed. Under these circumstances, GnRH antagonistic drugs which are expected to have substantially the same therapeutic effects as described above but not to cause the above-mentioned transient pituitary-gonadotropic action (acute action) have been desired. As compounds having such GnRH antagonistic activity, a number of compounds including, for example, derivatives of GnRH such as straight-chain peptides, (US Patent No. 5140009 and No. 5171835), cyclic hexapeptide derivatives (Japanese Patent Application Laid-open No. 61(1986)-191698] or bicyclic peptide derivatives [Journal of medicinal chemistry, Vol.36, pp.3265-3273, 1993] have been disclosed. These compounds are, however, all peptides, which leave many problems including, for example, dosage forms, stability of drugs, durability of actions and stability on metabolism. For solving these problems, orally administrable GnRH antagonistic drugs, especially non-peptide ones, are strongly desired. At the present stage, however, no report on non-peptide GnRH antagonistic drugs has been made.

The object of the invention lies in providing novel compounds having excellent gonadotropic hormone releasing hormone antagonistic activity as well as excellent gonadotropic hormone releasing hormone antagonistic agents.

EP 0443568 A1 discloses thienopyrimidine derivatives which differ structurally from the claim compounds in the substituents in position 1 which are used as angiotensine II antagonists.

DEOS 2 006 505 discloses isoflavonoid-ethers, which process gonadotropine inhibition activity.

EP 0640606 A1 discloses thienopyrimidine derivatives which differ structurally from the claim compounds in the substituents in position 3. This Reference is only prior art according to Article 54(3) EPC.

### Disclosure of Invention

Thus, the present invention provides
(1). A compound of the formula 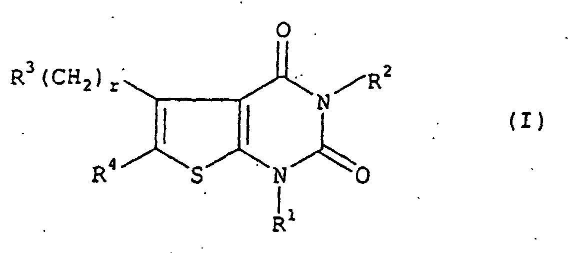 wherein
   - R¹: is C₆₋₁₄ aryl-methyl group which is substituted by 1 to 3 of
   (i) halogen,
   (ii) C₁₋₆ alkylthio and
   (iii) C₁₋₆ alkoxy;
   - R²: is
   (1) C₁₋₆ alkyl group which is unsubstituted or substituted by C₁₋₃ alkoxy,
   (2) C₆₋₁₄ aryl which is unsubstituted or substituted by 1 to 3 of (i) hydroxyl, (ii) C₁₋₆ alkoxy, (iii) a group of the formula: -S(O)ₙ-R⁶ in which n is an integer of 0 to 2 and R⁶ is C₁₋₃ alkyl and (iv) halogen,
   (3) C₇₋₁₀ aralkyl or
   (4) C₃₋₁₀ cycloalkyl;
   - R³: is a group of the formula: 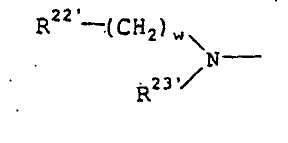 wherein
   R^{22'} is a phenyl or pyridyl, these groups being unsubstituted or substituted by a group of the formula: -S(O)ₙ-R⁶ in which n is an integer of 0 to 2 and R⁶ is C₁₋₃ alkyl,
   w is an integer of 0 to 3, and
   R^{23'} is a hydrogen or C₁₋₆ alkyl;
   - R⁴: is phenyl substituted by
   (1) amino,
   (2) C₁₋₈ acyl,
   (3) N-substituted C₁₋₇ alkylcarbamoyl,
   (4) nitro,
   (5) C₁₋₃ alkoxy which is unsubstituted or substituted by C₁₋₃ alkoxy,
   (6) a group of the formula: 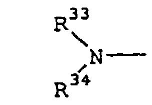 wherein
      R³³ is (i) C₁₋₆ alkyl, (ii) C₁₋₃ acyl which is unsubstituted or substituted by C₁₋₃ alkoxy, (iii) C₁₋₃ alkoxy which is unsubstituted or substituted by C₁₋₄ acyl, (iv) benzoyl or (v) formyl, and
      R³⁴ is (i) hydrogen or (ii) C₁₋₆ alkyl, or
   (7) C₂₋₄ alkenyl which is unsubstituted or substituted by C₁₋₃ alkoxy-carbonyl or C₁₋₃ alkyl-carbonyl; and
   - r: is 1;
   or 2,4(1H,3H)-dioxo-1-(2,6-difluorobenzyl)-6-(4-isobutyrylaminophenyl)-5-(N-benzyl-N-methylaminomethyl)-3-(3-methoxyphenyl)thieno[2,3-d]pyrimidine;
   or a salt thereof;
(2). A compound according to item (1), which is 2,4(1H,3H)-dioxo-6-(4-methoxyphenyl)-3-phenyl-1-(2-chloro-6-fluorobenzyl)-5-(N-benzyl-N-methylaminomethyl)thieno[2,3-d]pyrimidine or a salt thereof;
(3). A compound according to item (1), which is 2,4(1H,3H)-dioxo-1-(2,6-difluorobenzyl)-6-(4-propionylaminophenyl)-5-(N-benzyl-N-methylaminomethyl)-3-(3-methoxyphenyl)thieno[2,3-d]pyrimidine or a salt thereof;
(4). A method for producing a compound of the formula: 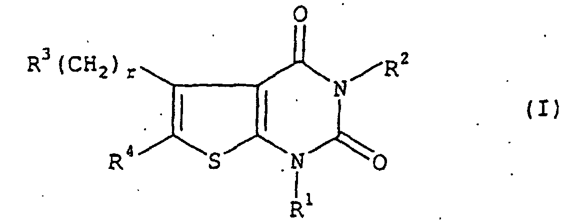 wherein R¹, R², R³, R⁴ and r are as defined in item (1) or a salt thereof, which comprises reacting a compound of the formula: 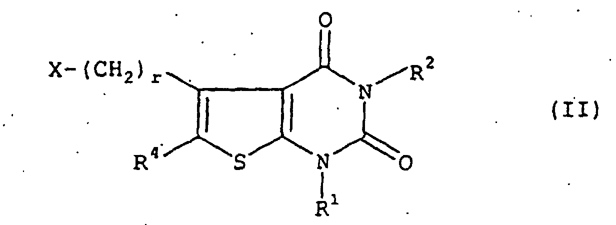 wherein R¹, R², R⁴ and r have the same meaning as defined above and X is a leaving group, or a salt thereof, with a compound of the formula:

   R³-H

   wherein R³ has the same meaning as defined above, or a salt thereof;
(5). A pharmaceutical composition, which comprises a compound as defined in item (1) and a carrier, excipient or diluent therefor;
(6). A composition according to item (5), which is a gonadotropin-releasing hormone antagonistic composition;
(7). A composition according to item (5), which is for preventing or treating a sex hormone dependent diseases;
(8). A compound according to item (1), which is for medicinal use;
(9). Use of a compound as defined in item (1) for producing a gonadotropin-releasing hormone antagonistic composition for antagonizing gonadotropin-releasing hormone in a mammal suffering from a gonadotropin-releasing hormone derived disorder;
(10). The use according to item (9), wherein the gonadotropin-releasing hormone derived disorder is a sex hormone dependent disease.

The nucleus of the present compound, 2,4(1H,3H)-dioxo- thieno[2,3-d]pyrimidine, is shown below;

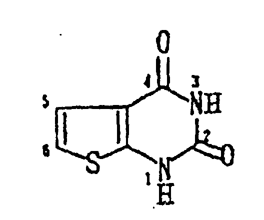

As the C₁₋₆ alkyl which may be substituted by C₁₋₃ alkoxy shown by R², mention is made of, for example, methyl, ethyl, propyl, isopropyl, butyl, sec-butyl, t-butyl, pentyl, hexyl. Among these, alkyl group having one to three carbon atoms is preferable.

As the C₆₋₁₄ aryl group shown by "C₆₋₁₄ aryl-methyl group" represented by R' or in the optionally substituted C₆₋₁₄ aryl group shown by R², mention is made of, for example, phenyl, naphthyl, anthryl, phenanthryl, acenaphthylenyl. Among these, phenyl, 1-naphthyl and 2-naphthyl are more preferable.

The number of substituents on the C₆₋₁₄ aryl group is one or more, preferably 1 to 3. Examples of the substituents on the C₆₋₁₄ aryl group shown by R² include (1) hydroxyl, (2) C₁₋₆ alkoxy (e.g. methoxy, ethoxy, propoxy, isopropoxy, butoxy, isobutoxy, sec-butoxy, t-butoxy, pentyloxy, hexyloxy) (3) a group of the formula: -S(O)n-R⁶ in which n is an integer of 0 to 2 and R⁶ is C₁₋₃ alkyl group (e.g. methylthio, ethylthio, propylthio, methylsulfinyl, ethylsulfinyl, propylsulfinyl, methylsulfonyl, ethylsulfonyl, propylsulfonyl), (4) halogen (e.g. fulorine, chlorine, bromine iodine).

As the C₃₋₁₀ cycloalkyl shown by R², mention is made of, for example, cyclopropyl, cyclobutyl, cyclopentyl, cyclohexyl, cycloheptyl, cyclooctyl. Among these, cyclopentyl and cyclohexyl are preferable.

As the C₇₋₁₀ aralkyl shown by R², mention is made of; for example, benzyl and phenethyl.

R¹ is made of C₆₋₁₄ aryl-methyl which may be substituted by halogen, C₁₋₆ alkylthio or C₁₋₆ alkoxy.

As further more preferred examples of the group R², mention is made of (1) a C₁₋₆ alkyl group which may be substituted by C₁₋₃ alkoxy, (2) a C₆₋₁₄ aryl group which may be substituted by 1 to 3 two of (i) hydroxy, (ii) C₁₋₆ alkoxy group, (iii) halogen and (iv) a group of the formula: -S(O)n-R⁶ in which n is an integer of 0 to 2 and R⁶ is a C₁₋₃ alkyl group, (3) C₇₋₁₀ aralkyl group or (4) a C₃₋₁₀ cycloalkyl group.

As more preferable examples of the group R², mention is made of (1) C₁₋₆ alkyl which may be substituted by C₁₋₃ alkoxy, (2) C₆₋₁₄ aryl which may be substituted by 1 to 3 of C₁₋₃ alkoxy and a group of the formula: -S(O)n-R⁶ wherein n is an integer of 0 to 2 and R⁶ is C₁₋₃ alkyl, or (3) C₃₋₁₀ cycloalkyl.

As the most preferred examples of the group R², mention is made of the C₆₋₁₄ aryl group which may be substituted by 1 to 3 of (1) a C₁₋₃ alkoxy group, (2) halogen and (3) a group of the formula: -S(O)n-R⁶ in which n is an integer of 0 to 2 and R⁶ is a C₁₋₃ alkyl group.

R³ is made of a group of the formula: 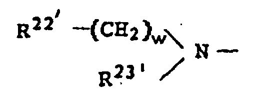 (wherein R^{22'} is phenyl or pyridyl, these groups being unsubstituted or substituted by a group of the formula: -S(O)ₙ-R⁶ in which n is an integer of 0 to 2 and R⁶ is a C₁₋₃ alkyl group, w is an integer of 0 to 3. R^{23'} is hydrogen or a C₁₋₆ alkyl group).

As examples of the substituents on phenyl shown by R⁴, mention is made of amino; C₁₋₈ acyl; N-substituted C₁₋₇ alkylcarbamoyl; nitro; C₁₋₃ alkoxy which may be substituted by C₁₋₃ alkoxy; a group of the formula; 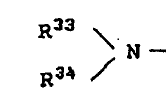 (wherein R³³ denotes C₁₋₆ alkyl, C₁₋₃ acyl which may be substituted by C₁₋₃ alkoxy; C₁₋₃ alkoxy which may be substituted by C₁₋₄ acyl; benzoyl; or formyl, R³⁴ denotes hydrogen or C₁₋₆ alkyl), C₂₋₄ alkenyl which may be substituted by C₁₋₃ alkoxy-carbonyl or C₁₋₃ alkyl-carbonyl.

As C₁₋₆ alkyl in the above definition, mention is made of, for example, methyl, ethyl, propyl, isopropyl, butyl, isobutyl, sec-butyl, t-butyl, pentyl, isopentyl, neopentyl and hexyl.

As the C₁₋₈ acyl, mention is made of for example C₁₋₈ alkanoyl (formyl), C₆ arylcarbonyl (benzoyl), C₇ aralkyl-carbonyl (benzylcarbonyl) and C₇ aralkyloxy-carbonyl (benzyloxycarbonyl).

As the preferable C₁₋₈ acyl and C₁₋₈ alkanoyl in the above definition, mention is made of C₁₋₆ alkyl-carbonyl, and C₁₋₆ alkyl is of the same meaning as defined above.

As C₁₋₆ alkoxy in the above definition, is made of for example methoxy, ethoxy, propoxy, isopropoxy, butoxy, isobutoxy, sec-butoxy, t-butoxy, pentyloxy, isopentyloxy, neopentyloxy, hexyloxy. Among these, alkoxy having 1 to 3 carbon atoms is preferable.

As C₂₋₄ alkenyl in the above definition, is made of for example vinyl, allyl, 1-butenyl, 2-butenyl.

Examples of the C₆₋₁₄ aryl includes phenyl, naphthyl.

Examples of C₇₋₁₀ aralkyl includes benzyl, phenethyl.

As the halogen, mention is made of fluorine, chlorine, bromine, iodine.

The compounds (I) of the present invention can be produced easily by per se known methods, as exemplified by the following production methods, or a similar method thereto.
1. Method A: In accordance with the method disclosed by K. Gewald, E. Schinke and H. Bøttcher, Chem. Ber., 99, 94-100 (1966), an adequate ketone or aldehyde having an active methylene (i) is allowed to react with a cyanoacetic acid ester derivative and sulfur to convert into a 2-aminothiophene derivative (ii). More specifically, in the case of using ketone (R^{3'}≠H), it is subjected to heating under reflux together with a cyanoacetic acid ester.derivative, in the presence of acetic acid and ammonium acetate, in a proper solvent such as toluene to give an alkylidene cyanoacetic acid ester derivative, which is then heated in an adequate solvent, for example, ethanol in the presence of sulfur and a base to afford a 2-aminothiophene derivative (ii). And, in the case of using aldehyde (R^{3'}=H), it is heated in a proper solvent, for example, dimethylformamide, in the presence of a cyanoacetic acid ester derivative, sulfur and a base to give a 2-aminothiophene derivative (ii).

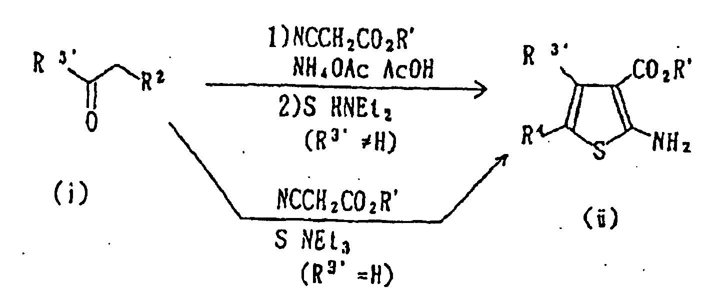

The 2-aminothiophene derivative (ii) produced by the method described in Production Method 1 or a salt thereof is allowed to react with an isocyanate derivative. The isocyanate derivative is exemplified by derivatives represented by the formula, R²-NCO (wherein R² is of the same meaning as defined above). The reaction of the compound (ii) or a salt thereof with the isocyanate derivative is conducted in an solvent which does not adversely affect the reaction (e.g. tetrahydrofuran, pyridine, dioxane, benzene, dichloromethane, 1,2-dichloroethane, toluene, xylene) at temperatures ranging from about 15 to about 130°C. The isocyanate derivative is employed in an amount of about 1 to 5 equivalents, preferably about 1.1 to 2.5 equivalents, relative to 1 equivalent of the compound (ii). The reaction-time ranges from several hours to several days, preferably from about 15 minutes to about two days.
2. Method B: Amine [e.g. a compound represented by the formula R²-NH₂ (wherein R² is of the same meaning as defined above)] is subjected to addition reaction to an isocyanate derivative produced by allowing a 2-aminothiophene derivative (ii) or a salt thereof to react with phosgene or an equivalent compound thereof [e.g. diphosgene such as bis(trichloromethyl)carbonate, triphosgene such as trichloromethylchloroformate]. The reaction of the compound (ii) or a salt thereof with phosgene or an equivalent compound thereof is conducted in a solvent which does not affect adversely the reaction (e.g. dioxane, tetrahydrofuran, benzene, toluene, xylene, 1,2-dichloroethane, chloroform) at temperatures ranging from about 40 to 120°C. Phosgene or an equivalent compound thereof is employed in an amount ranging from about 0.5 to 2 equivalents, preferably from about 0.9 to 1.1 equivalent). The reaction time ranges from several minutes to several days, preferably from about 15 minutes to about two days. The addition reaction of amine is conducted in a solvent which does not affect adversely the reaction (e.g. pyridine, tetrahydrofuran, dioxane, benzene, dichloromethane, 1,2-dichloroethane, toluene, xylene) at temperatures ranging from about 15 to 130°C. Amine is employed in an amount ranging from about 1 to 5 equivalents, preferably from about 1.1 to 3 equivalents. The reaction time ranges from several minutes to several days, preferably from about 15 minutes to about two days.

The compound (XV) or a salt thereof thus produced is processed with a base to cause ring-closure reaction to thereby produce a thieno [2,3-d] pyrimidine derivative (XVI). The ring-closure reaction is conducted in a solvent which does not affect adversely the reaction. The solvent is exemplified by alcohols such as methanol, ethanol or propanol, and ethers such as dioxane or tetrahydrofuran. As the base, use is made of, for example, an alkali metal alkoxide such as sodium methylate, sodium ethylate or sodium isopropoxide, and an alkali metal hydride such as sodium hydride. The amount of the base to be employed ranges from 1 to 5 equivalents, preferably from about 1.5 to 3 equivalents, relative to 1 equivalent of the compound (XV). The reaction temperature ranges from about 10°C to the boiling point of the solvent then employed, preferably from about 25°C to the boiling point of the solvent then employed. The reaction time ranges from several minutes to several days, preferably from about 10 minutes to two days.

The compound (XVI) and a halogenated aralkyl derivative are stirred, in the presence of a base (e.g. an organic base such as pyridine or triethylamine), in a solvent which does not affect adversely the reaction (e.g. amides such as dimethylformamide or dimethylacetamide), at about 10 to 100°C, to produce a 2,4-dioxothieno[2,3-d]pyrimidine derivative (IIa). Subsequently, the compound (IIa) is stirred together with N-bromosuccinimide (NBS) in a solvent.which does not affect adversely the reaction (e.g. halogenated hydrocarbons such as carbon tetrachloride or chloroform), in the presence of α, α'-azobisisobutyronitrile, to thereby produce the compound (II). Further, the compound (II) is stirred together with various amines, in the presence of a base, in a solvent which does not affect adversely the reaction (e.g. amides such as dimethylformamide or dimethylacetamide, nitriles such as acetonitrile, alcohols such as ethanol), at temperatures ranging from about 10 to 100°C for 0.5 to 8 hours, to thereby produce the compound (I). When necessary, the compound (I) is made into a corresponding salt with a suitable acid (e.g. hydrochloric acid or oxalic acid).

The foregoing production method is shown by the following scheme 1:

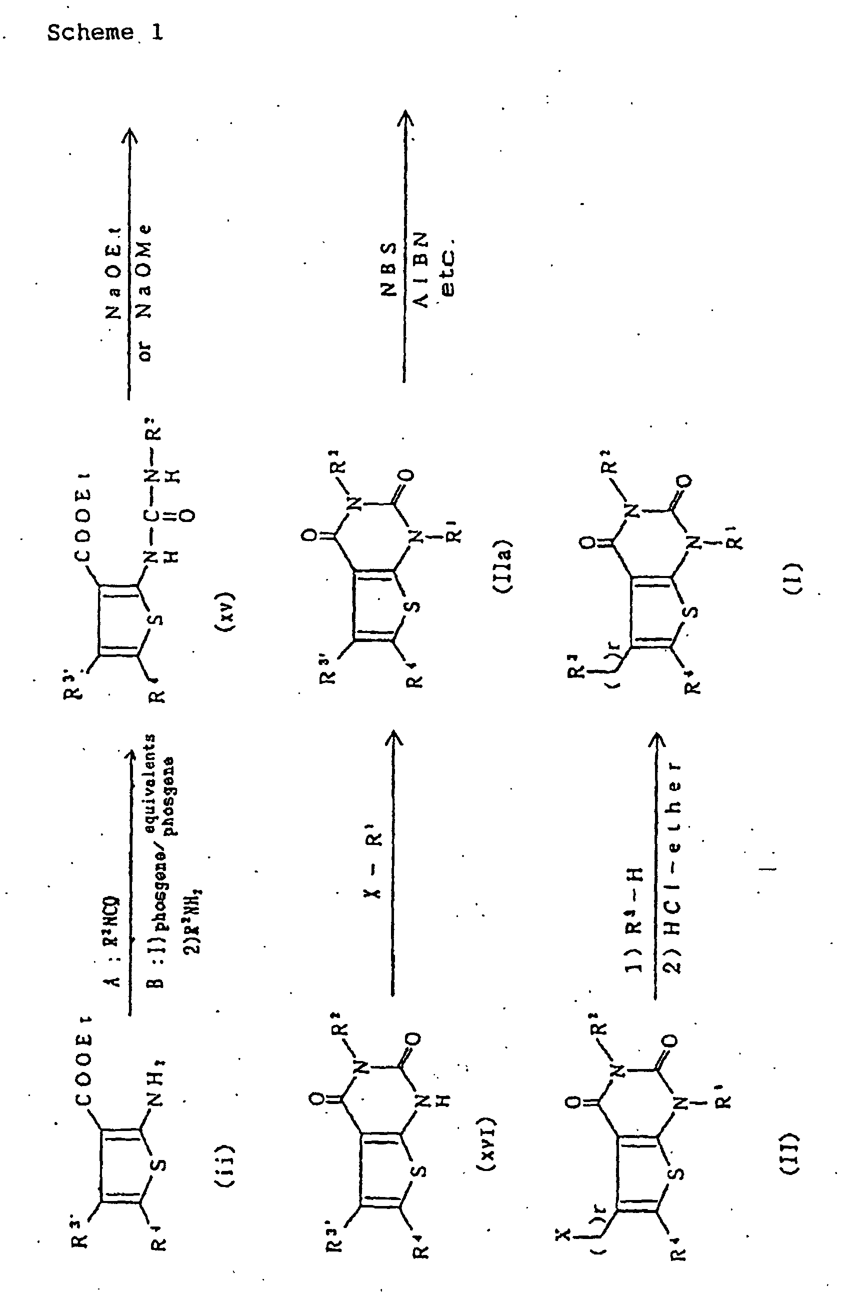

The respective groups described in the above scheme have the same meaning as defined above. X denotes a leaving group.

As the leaving group shown by the above X, mention is made of, for example, groups readily susceptible to substitution reaction by a nucleophilic reagent (e.g. the hydrocarbon residue having a hetero-atom with negative electric charge (e.g. oxygen atom,sulfur atom and nitrogen atom)). More specifically, for example, halogen atom (e.g. iodide, bromide, chloride), alkanoyloxy (e.g. acetoxy, alkylsulfonyloxy (e.g. methanesulfonyloxy) and alkyl-aryl sulfonyloxy (e.g. p-toluenesulfonyloxy) are mentioned.
3. Method C: In place of the production method from the compound (ii) to the compound (IIa) in the above scheme 1, any per se conventional methods can be employed for example the following processes for producing the compound (IIa) from the compound (ii). Namely, the compound (ii) is dissolved in an appropriate solvent, e.g. methanol, ethanol, which does not adversely affect the reaction, 2N sodium hydroxide is added, and the mixture is reacted at room tempereture to heating (till about 100°C) for one to 12 hours. The obtained. compound wherein -COOEt is converted to -COOH is dissolved in an appropriate solvent, e.g. dioxane, and to the solution is added an equivalent amount of triphosgene and the mixture is reacted at a temperature of 80 to 150°C for one to 10 hours under stirring. The obtained 1-hydroxy oxazine compound is treated in a manner similar to that of the reaction of the compound (XVI) to the compound (IIa) as mentioned above. Thus obtained oxazine compound to which the group R¹ is introduced at 1-position is dissolved in an appropriate solvent, e.g. dichloromethane, to the solution is added an equivalent amount to a small excess amount of an amine, e.g. ammonium, alkylamine, arylamine, and the mixture is reacted at a room temperature to heating (till 100°C) for 1 to 12 hours under stirring. Then, to the reaction mixture is added triphosgene again and triethylamine as a base, the mixture is reacted at about 100°C under reflux for 1 to 6 hours, to give a compound of the formula (IIa).
4. Other methods:

The substituents on the compound (I) can be converted to other substituents by per se known and conventional methods. Examples of the methods are shown below.
(i) The nitro group as the substituent can be converted to an amino group when the starting compound is dissolved in an appropriate solvent, e.g. ethanol, methanol, and (a) to the solution is added palladium-carbon, and the mixture is reacted at room temperature for one to 12 hours under the hydrogen atmosphere, or (b) to the solution is added iron powder and hydrochloric acid, and the mixture is reacted at room temperature for one to 12 hours.
(ii) The amino group can be converted to an acylated amino group in that the starting compound is dissolved in an appropriate solvent, e.g. tetrahydrofuran, dimethylsulfoxide, to the solution is added potassium carbonate, pyridine and triethylamine as a base and acid anhydride or acid halide. The mixture is reacted at a room temperature for one to 10 hours under stirring.
(iii) From an amino compound, a compound having the amino group is converted to alkenyl-amino compound. For example, the starting compound is dissolved in an appropriate solvent, e.g. acetic acid, dimethylformamide, dichloromethane, tetrahydrofuran, dioxane, acetonitrile, to the solution is added diazonizing agent, e.g. sodium nitrite, isoamyl nitrite, to the mixture is added palladium catalyst, e.g. bis(dibenzylideneacetone)palladium and one to excess equivalents of alkenyl derivative, and the mixture is stirred at room temperature to heating (80°C) for one to 12 hours.
(iv) A carbon atom can be introduced to the amino group, for example, to the starting compound in an appropriate solvent, e.g. acetic acid, dimethylformamide, dichloromethane, tetrahydrofuran, dioxane, is added an acrylic acid derivative or oxirane derivative, e.g. epoxide compound. The mixture is stirred at 0 to 80°C for 6 to 24 hours.
(v) A sulfur atom can be introduced to the amino group in the compound, for example, to the starting compound in an appropriate solvent, e.g. pyridine, dimethylformamide, dichloromethane, tetrahydrofuran, ethylether, dioxane, is added halide of sulfur compound. The mixture is stirred at 0 to 80°C for 6 to 24 hours.
(vi) The substituent, formyl group, can be converted to methyl group in that a starting compound is dissolved in an appropriate solvent, e.g. tetrahydrofuran, and to the mixture is added an organic borane, derivative, e.g. dimethylsulfide borane, and the mixture is reacted at room temperature to heating under reflux for a several hours, e.g. one to 3 hours.
(vii) From methoxy derivative, actonyloxy derivative can be prepared in that the starting material is dissolved in an appropriate solvent, e.g. dichloromethane, to the solution is added one to excess equivalents of Lewis acid, e.g. aluminium chloride, and thiol compound or sulfide compound (e.g. dimethylsulfide), and the mixture is reacted at ice-cooling to room temperature for one to 10 hours, and then the obtained hydroxy derivative is dissolved in an appropriate solvent, e.g. dimethylformamide, to the solution is added a base, e.g. sodium hydroxide or potassium carbonate, and an alkyl halide. The mixture is reacted at a room temperature for one to 12 hours.
(viii) A group of methoxy can be changed to isopropoxy in that the starting material is dissolved in an appropriate solvent, e.g. dichloromethane, to the solution is added one to excess equivalents of Lewis acid, e.g. aluminum chloride, and thiol compound or sulfide compound, e.g. dimethylsulfide, and the mixture is reacted at room temperature to ice-cooling for one to 10 hours.
(ix) An aminocarbonyl group can be introduced in that a starting compound having halogen atom is dissolved in an appropriate solvent, e.g. dimethoxyethane, to the solution is added arylborric acid derivative, a base, e.g. sodium carbonate, a palladium compound e.g. tetrakis(triphenylphosphine)palladium(0), as a catalyst and the mixture is refluxed 1 to 6 hours.
(x) An alkylthio compound can be converted to an alkylsulfinyl compound or an alkylsulfonyl compound by reacting a starting compound with an oxidizing agent, e.g. metachloroperbenzoic acid, in an appropriate solvent, e.g. dichloromethane, at ice-cooling to heating. When heating harder or treating with an excess amount of oxidizing agent, an alkylsulfonyl compound is obtained.

As salts of the compounds (I) of this invention obtained thus above, physiologically acceptable acid addition salts are preferable. Examples of such salts include those with an inorganic acid (e.g. hydrochloric acid, hydrobromic acid, nitric acid, sulfuric acid and phosphoric acid) or those with an organic acid (e.g. formic acid, acetic acid, trifluoroacetic acid, fumaric acid, oxalic acid, tartaric acid, maleic acid, citric acid, succinic acid, malic acid, methanesulfonic acid, bezenesulfonic acid, and p-toluenesulfonic acid).
Further, when the compound (I) of this invention has an acid group such as -COOH, the compound(I) may form a salt with an inorganic base (e.g. an alkali metal or alkaline earth metal such as sodium, potassium, calcium and magnesium; ammonia) or an organic base (e.g. trimethylamine, triethylamine, pyridine, picolin, ethanolamine, diethanolamine, triethanolamine, dicyclohexylamine and N,N'-dibenzylethylenediamine).

The compounds (I) or salts thereof of the present invention produced above can be isolated and purified by a conventional separating means such as recrystallization, distillation and chromatography. In the case where the compound (I) is produced in the free form, it can be converted to a salt thereof by a per se conventional means or a method analogous thereto. On the contrary, when it is obtained in the form of a salt, it can be converted to its free form or to any other salt.

In the case where the compound (I) or a salt thereof of the present invention is an optically active compound, it can be separated into d-form and 1-form by means of a conventional optical resolution.

Since the compounds (I) of this invention have a GnRH antagonistic activity and low in toxicity, they can be safely used for the therapy of male hormone or female hormone dependent diseases as well as the therapy of diseases caused by excess secretion of these hormones, in mammalian animals (e.g. human, monkey, cow, horse, dog, cat, rabbit, rat, mouse, etc.), suppressing the secretion of gonadotropic hormone by the action of GnRH receptor antagonistic action. More specifically, the compounds of this invention are effective as a prophylactic or therapeutic agent for the prevention or treatment of several hormone dependent diseases, for example, a sex hormone dependent cancer (e.g. prostate cancer, cancer of the uterine cervix, breast cancer, pituitary adenoma), benign prostatic hypertrophy, myoma of the uterus, endometriosis, precocious puberty, amenorrhea, premenstrual syndrome, polycystic ovary syndrome and acne vulgaris. And, the compounds of this invention are also effective as a fertility controlling agent in both sexes (e.g. pregnancy controlling agents and menstrual cycle controlling agents). The compounds of this invention can be further used as a contraceptive of male or female and, as an ovulation-inducing agent of female. The compound of this invention can be used as an infertility treating agent by using a rebound effect owing to a stoppage of administration thereof. Further, the compounds of this invention are useful as modulating estrous cycles in animals in the field of animal husbandry, and as an agent for improving the quality of edible meat or promoting the growth of animals. Besides, the compounds of this invention are useful as an agent of spawning promotion in fish. While the compounds of this invention can be used singly, they can also effectively be used by administering in combination with a steroidal or non-steroidal antiandrogenic agent. The compound of this invention can be used for the suppressing a passing ascent of testosterone concentration in plasma, the ascent which occurs in administration of GnRH super antagonist such as leuprorelin acetate. The compound of this invention can effectively be used by administering in combination with a chemoterapeutic agent for cancer. In treatment of prostate cancer, examples of the chemoterapeutic agent include Ifosfamide, UFT, Adriamycin, Peplomycin, Cisplatin and the like. In treatment of breast cancer, examples of the chemoterpeutic agent include Cyclophohamide, 5-FU-, UFT, Methotrexate, Adriamycin, Mitomycin C, . Mitoxantrone and the like.

The present compounds (I) shows sufficient GnRH activity through subcutaneous or oral administration, is stably absorbed through oral administration and shows GnRH activity over a long time.

When the compound (I) of this invention is employed, in the field of animal husbandry or fisheries, as prophylactic and therapeutic agents of the above-mentioned diseases, it can be administered orally or non-orally in accordance with per se known means. It is mixed with a pharmaceutically acceptable carrier and usually administered orally as a solid preparation such as tablet, capsule, granule or powder, or non-orally as intravenous, subcutaneous or intramuscular injection, or as suppository or sublingually administrable tablet. Further, it is sublingually, subcutaneously or intramuscularly administered as a prolonged release formulation such as sublingually administrable tablets, or microcapsules. The daily dose of the present compound (I) varies with the degree of affliction; age, sex, body weight and difference of sensitivity of the subject to be administered; the time and intervals of administration, properties, dosage forms and kinds of the medicinal preparation; and kinds of the effective components, and it ranges usually, though not specifically limited, from about 0.01 to 10 mg, preferably from about 0.02 to 2 mg, more preferably from about 0.01 to 1 mg, relative to 1 kg body weight of mammalian animals, which is administered usually once daily or by 2 to 4 divided dosages. The daily dose when used in the field of animal husbandry or fishery varies with the conditions analogous to those mentioned above, it ranges, relative to 1 kg body weight of the subject animal or fish, from about 0.001 to 5 mg, preferably from about 0.002 to 2 mg, once or 2 to 3 divided dosages.

As the above-mentioned pharmaceutically acceptable carriers, conventional various organic or inorganic carriers are used, and they are incorporated as excipients, lubricants, binders and disintegrants in solid compositions; and as solvents, solubilisers, suspending agents, isotonizing agents, buffering agents and pain-easing agents in liquid compositions. And, depending on necessity, further additives such as preservatives, anti-oxidants,. coloring agents and sweeteners can also be used.

Preferable examples of the above-mentioned excipients include lactose, sugar, D-mannitol, starch, crystalline cellulose and more volatile silicon dioxide. Preferable examples of above-mentioned lubricants include magnesium stearate, calcium stearate, talc and colloid silica. Preferable examples of the above-mentioned binders include crystalline cellulose, sugar, D-mannitol, dextrin, hydroxypropyl cellulose, hydroxymethyl cellulose and polyvinyl pyrrolidone. Preferable examples of the above-mentioned disintegrants include starch, carboxymethyl cellulose, carboxymethyl cellulose calcium, cross carmelose sodium, cross carmelose sodium and carboxymethyl starch sodium. Preferable examples of the above-mentioned solvents include water for injection, alcohol, propylene glycol, macrogol, sesame oil and corn oil. Preferable examples.of the above-mentioned solubilizers include polyethylene glycol, propylene glycol, D-mannitol, benzyl benzoate, ethanol, tris-aminomethane, cholesterol, triethanolamine, sodium carbonate and sodium citrate. Preferable examples of the above-mentioned suspending agents include surfactants such as stearyl triethanolamine, sodium lauryl sulfate, lauryl aminopropionic acid, lecithin, benzalkonium chloride, benzetonium chloride and monostearic glyceryl ester; and hydrophilic polymers such as polyvinyl alcohol, polyvinyl pyrrolidone, sodium carboxymethyl cellulose, methyl cellulose, hydroxymethyl cellulose, hydroxyethyl cellulose and hydroxypropyl cellulose. Preferable examples of the above-mentioned isotonizing agents include sodium chloride, glycerin and D-mannitol. Preferable examples of the above-mentioned buffering agents include buffer solutions such as phosphate, acetate, carbonate and citrate. Preferable examples of the above-mentioned pain-easing agents include benzyl alcohol. Preferable examples of the above-mentioned preservatives include para-hydroxybenzoic acid esters, chlorobutanol, benzyl alcohol, phenethyl alcohol, dehydroacetic acid and sorbic acid. Preferable examples of the above-mentioned anti-oxidants include sulfite and ascorbic acid.

To the compound (I) of this invention, are added, for example, a suspending agent, a solubilizer, a stabilizer, an isotonizing agent and a preservative, then the mixture is formulated, in accordance with a per se known method, into an intravenous, subcutaneous or intramuscular injection. These injections can be processed into lyophilized preparations, when necessary, by a per se known method.

Examples of the above-mentioned pharmaceutical composition are oral agents (e.g. diluted powders, granules, capsules and tablets), injections, dropping injections, external agents (e.g. transnasal preparations, percutaneous preparations, etc.), ointments (e.g. rectal ointment, vaginal ointment, etc.) and the like.

Such pharmaceutical compositions can be manufactured by a per se known method commonly used in preparing pharmaceutical compositions.

The compound (I) of the present invention or a salt thereof can be made into injections either in a form of an aqueous injection together with dispersing agents [e.g. Tween 80 (Atlas Powder, U.S.A.), HCO 80 (Nikko Chemicals, Japan), polyethylene glycol, carboxymethylcellulose, sodium alginate, etc.], preservatives (e.g. methyl paraben, propyl paraben, benzyl alcohol, etc.), isotonizing agents (e.g. sodium chloride, mannitol, sorbitol, glucose, etc.) and the like or in a form of an oily injection by dissolving, suspending or emulsifying in plant oil (e.g. olive oil, sesame oil, cotton seed oil, corn oil, etc.), propylene glycol and the like.

In preparing a pharmaceutical composition for oral use, the compound (I) of the present invention or a salt thereof is molded by compressing, for example, with fillers (e.g. lactose, sucrose, starch, etc.), disintegrating agents (e.g. starch, calcium carbonate, etc.), binders (e.g. starch, gum arabic, carboxymethylcellulose, polyvinylpyrrolidone, hydroxypropylcellulose, etc.) or lubricants (e.g. talc, magnesium stearate, polyethylene glycol 6000, etc.) and the like. If necessary, the composition is coated by a per se known method with an object of masking the taste, enteric coating or long-acting. Examples of the coating agent therefore are hydroxypropylmethylcellulose, ethylcellulose, hydroxymethylcellulose, hydroxypropylcellulose, polyoxyethylene glycol, Tween 80, pluronic F 68, cellulose acetate phthalate, hydroxypropylmethylcellulose phthalate, hydroxymethylcellulose acetate succinate, Eudragit (a copolymer of methacrylic acid with acrylic acid; manufactured by Rohm, Germany), red oxide of iron and the like. Subcoating layer may be provided between the enteric coating and the core according to per se known method.

In preparing an external composition, the compound (I) of the present invention or a salt thereof as it is or a salt thereof is subjected to a per se known method to give a solid, semisolid or liquid agent for external use. For example, the solid preparation is manufactured as follows. Thus, the compound of the present invention as it is or after adding/mixing fillers (e.g. glycol, mannitol, starch, microcrystalline cullulose, etc.), thickeners (e.g. natural gums, cellulose derivatives, acrylic acid polymers, etc.) and the like thereto/therewith is made into a powdery composition. With respect to the liquid composition, an oily or aqueous suspension is manufactured by the manner nearly the same as in the case of the injection. In the case of a semisolid composition, the preferred one is an aqueous or oily gel or an ointment. Each of them may be compounded with a pH adjusting agent (e.g. carbonic acid, phosphoric acid, citric acid, hydrochloric acid, sodium hydroxide, etc.), an antiseptic agent (e.g p-hydroxybenzoates, chlorobutanol, benzalkonium chloride, etc.) and the like.

In the manufacture of an ointment for example, the compound (I) of the present invention or a salt thereof can be made into an oily or an aqueous solid, semisolid or liquid ointment. Examples of the oily base material applicable in the above-mentioned composition are glycerides of higher fatty acids [e.g. cacao butter, Witepsols (manufactured by Dynamite-Nobel), etc.], medium fatty acids [e.g. Miglyols (manufactured by Dynamite-Nobel), etc.] and plant oil (e.g. sesame oil, soybean oil, cotton seed oil, etc.) and the like. Examples of the aqueous base material are polyethylene glycols and propylene glycol and those of the base material for aqueous gel are natural gums, cellulose derivatives, vinyl polymers, acrylic acid polymers, etc.

### Best Mode for Carrying Out of the Invention

By way of the following Reference Examples and Working Examples, the present invention will be described more specifically, but they are not intended to limit the scope of this invention thereto.

¹H-NMR spectra were taken with the Varian GEMINI 200 (200 MHz) type spectrometer, JEOL LAMBDA300 (300MHz) type spectrometer or the Brucker AM 500 (500 MHz) type spectrometer, employing tetramethylsilane as the internal standard. All delta values were expressed in ppm.

The symbols used in the present specification have the following meanings:
s: singlet, d: doublet, t: triplet, dt: double triplet, m: multiplet, br: broad

### Reference Example 1

### Production of 2-amino-5-phenylthiophene-3-carboxylic acid ethyl ester:

To a mixture of ethyl cyanoacetate (6.1 g, 50 mmol), sulfur (1.61 g, 50 mmol) triethylamine (3.5 ml, 25 mmol) and dimethylformamide (10 ml) was added dropwise, with stirring at 45°C, phenylacetaldehyde (50% diethylphthalate solution; 12.05 g, 50 mmol) for 20 minutes. The mixture was stirred for 9 hours at 45°C, and the reaction mixture was concentrated. The resulting residue was extracted with ethylacetate. The extract was washed with an aqueous sodium chloride solution, which was then dried (MgSO₄), followed by distilling off the solvent under reduced pressure. The residue was chromatographed on silica gel, followed by crystallization from ether-hexane to give slightly yellow plates (5.55 g, 45%), m.p.124.5-125.5°C (value in literature reference 123-124°C).

| Elemental Analysis for C₁₃H₁₃NO₂S: | | | |
|---|---|---|---|
| | C(%) | H(%) | N(%) |
| Calcd. | 63.13 ; | 5.30 ; | 5.66 |
| Found | 62.99 ; | 5.05 ; | 5.63 |

¹H-NMR (200MHz, CDCl₃) δ: 1.37(3H,t,J=7.1Hz), 4.30(2H,d,J=7.1Hz), 5.97(2H,br), 7.17-7.46(6H,m). IR(KBr): 3448, 3320, 1667, 1590, 1549 cm⁻¹.

### Reference Example 2

### Production of 2-amino-4-methyl-5-(4-methoxyphenyl)thiophene-3-carboxylic acid ethyl ester:

A mixture of 4-methoxyphenylacetbne (16.5 g, 0.10 mol), ethyl cyanoacetate (12.2 g, 0.10 mol), ammonium acetate (1.55 g, 20 mmol), acetic acid (4.6 ml, 80 mmol) and benzene (20 ml) was heated for 24 hours under reflux, while removing water produced in the reaction mixture using a Dean and Stark apparatus. After cooling, the reaction mixture was concentrated under reduced pressure. The residue was partitioned between dichloromethane and an aqueous sodium hydrogencarbonate solution. The organic layer was washed with an aqueous sodium chloride solution, which was then dried (MgSO₄), followed by distilling of the solvent under reduced pressure. To an ethanol (30 ml) solution of the residue were added sulfur (3.21 g, 0.10 mol) and diethylamine (10.4 ml, 0.10 mol). The mixture was stirred at 50-60°C for 2h and then concentrated, and the concentrate was extracted with ethyl acetate. The extract was washed with an aqueous sodium chloride solution and dried (MgSO₄), followed by distilling off the solvent under reduced pressure. The residue was chromatographed on silica gel, which was the crystallized from ether-hexane to give a pale yellow plates (11.5 g, 40%), m.p.79-80°C.

| Elemental Analysis for C₁₅H₁₇NO₃S: | | | | |
|---|---|---|---|---|
| | C(%) | H(%) | N(%) | S(%) |
| Calcd. | 61.83 ; | 5.88 ; | 4.81 ; | 11.01 |
| Found | 61.81 ; | 5.75 ; | 4.74 ; | 10.82 |

¹H-NMR (200MHz, CDCl₃) δ: 1.37(3H,t,J=7.1Hz), 2.28(3H,s), 3.83(3H,s), 4.31(2H,q,J=7.1Hz), 6.05(2H,brs), 6.91(2H,d,J=8.8Hz), 7.27(2H,d,J=8.8Hz).
IR(KBr): 3426, 3328, 1651, 1586, 1550, 1505, 1485 cm⁻¹.
FAB-MS m/z: 291 (M⁺)

### Reference Example 3

Employing various acetone derivatives in place of 4-methoxyphenylacetone, compounds shown in Table 1 are produced in accordance with substantially the same manner as described in Reference Example 2.

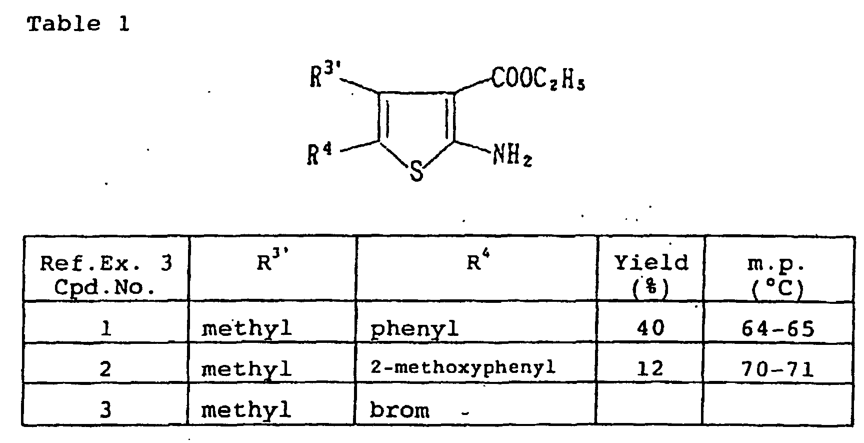

### Reference Example 4

### Production of 2-amino-4-methyl-5-(4-nitrophenyl)thiophene-3-carboxylic acid ethyl ester:

In substantially the same procedure as described in Reference Example 2, using 4-nitrophenylacetone (35.0 g, 195 mmol) in place of 4-methoxyphenyl acetone, ethyl cyanoacetate (23 g, 19.5 mmol), ammonium acetate (3.1 g, 40 mmol), acetic acid (9.1 ml, 159 mmol), sulfur (5.0 g, 160 mmol) and diethylamine (16.0 ml, 160 mmol), the titled compound was produced as colorless crystals (22.2 g, 52%). m.p.168-170°C (recrystallized from ether-hexane).

| Elemental Analysis for C₁₄H₁₄N₂O₄S: | | | |
|---|---|---|---|
| | C(%) | H(%) | N(%) |
| Calcd. | 54.89 ; | 4.61 ; | 9.14 |
| Found | 54.83 ; | 4.90 ; | 9.09 |

¹H-NMR (200MHz, CDCl₃) δ: 1.39(3H,t,J=7.1Hz), 2.40(3H,s), 4.34(2H,q,J=7.1Hz), 6.27(2H,brs),
7.48(2H,d,J=8.7Hz), 8.23(2H,d,J=8.7Hz).
IR (KBr): 3446, 3324, 1667, 1580, 1545, 1506, 1491, 1475, 1410, 1332 cm⁻¹.

### Reference Example 5

### Production of 2,4(1H,3H)-dioxo-5-methyl-6-(4-methoxyphenyl)-thieno[2,3-d]pyrimidin-3-acetic acid ethyl ester:

To a solution of the compound produced in Reference Example 2 (5.00 g, 17.20 mmol) was added ethyl isocyanatoacetate (2.90 ml, 25.80 mmol). The mixture was stirred for 6 hours at 45°C, followed by concentration under reduced pressure. The concentrate was dissolved in ethanol (6 ml), to which was added sodium ethoxide {prepared from ethanol (30 ml) and sodium (0.79 g, 34.30 mmol)}. The mixture was stirred for 24 hours at room temperature, to which was added 2N HCl (18 ml, 36 mmol). Ethanol was distilled off under reduced pressure, and the residue was subjected to filtration, which was washed with water-ethanol and dried under reduced pressure, followed by recrystallization from ethanol to give white needles (5.70 g, 89%). m.p.164-165°C.

| Elemental Analysis for C₁₈H₁₈N₂O₅S: | | | |
|---|---|---|---|
| | C(%) | H(%) | N(%) |
| Calcd. | 57.74 ; | 4.85 ; | 7.48 |
| Found | 57.78 ; | 5.03 ; | 7.45 |

¹H-NMR (200MHz, CDCl₃) δ: 1.30(3H,t,J=7.2Hz), 2.45(3H,s), 3.85(3H,s), 4.26(2H,q,J=7.2Hz), 4.78(2H,s), 6.95(2H,d,J=8.8Hz), 7.31(2H,d,J=8.8Hz), 10.58(1H,s).
IR (KBr): 2914, 1742, 1713, 1655, 1605, 1568, 1528, 1499 cm⁻¹.

### Reference Example 6

Employing, as starting materials, the compounds which are produced in Reference Examples 2, 3 or 4, compounds which are produced in accordance with the method described in Reference Example 5 are set forth in Table 2.

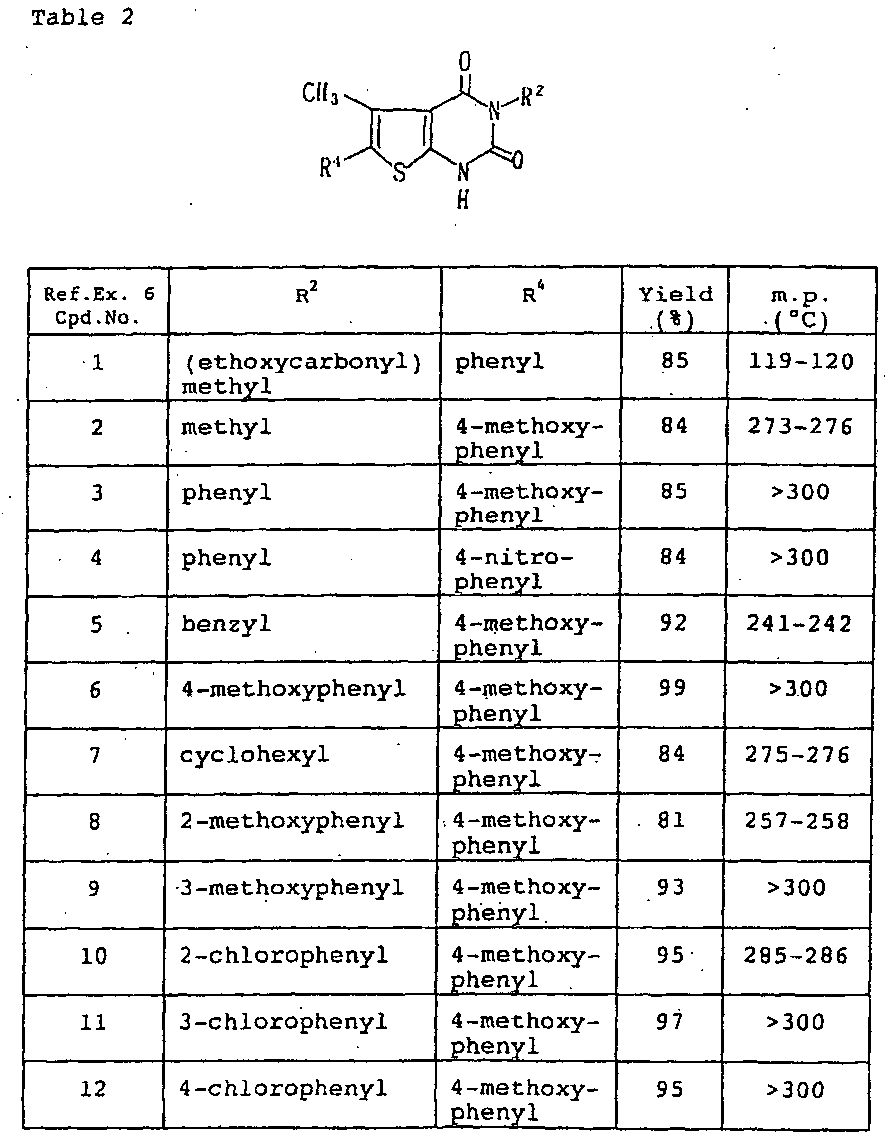

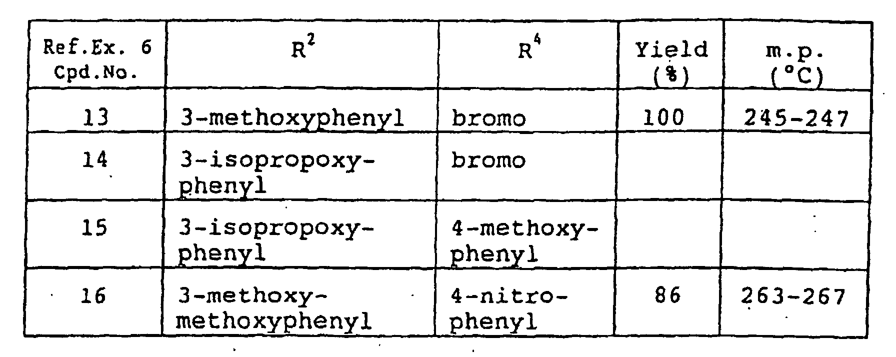

### Reference Example 7

### Production of 2,4(1H,3H)-dioxo-6-(4-nitrophenyl)-5-methylthieno[2,3-d]pyrimidin-3-acetic acid ethyl ester:

To the compound 1 produced in Reference Example 6 (2.20 g, 6.39 mmol) was added conc, sulfuric acid (12 ml). To the mixture was added dropwise, under ice-cooling, a solution of sodium nitrate (550 mg, 6.47 mmol) in conc. sulfuric acid, followed by stirring for one hour under ice-cooling. The reaction mixture was poured into ice-water, which was extracted with ethyl acetate. The extract was washed with an aqueous sodium chloride solution and dried (MgSO₄), followed by distilling off the solvent under reduced pressure. The residue was chromatographed on silica gel to give a yellowish solid (1.30 g, 52%), which was then recrystallized.from ethyl acetate - hexane to yellow crystals, m.p.277-280°C.

| Elemental Analysis for C₁₇H₁₅N₃O₆S·0.4H₂O: | | | |
|---|---|---|---|
| | C(%) | H(%) | N(%) |
| Calcd. | 51.48 ; | 4.01 ; | 10.59 |
| Found | 51.64 ; | 3.79 ; | 10.61 |

¹H-NMR (200MHz, CDCl₃) δ: 1.33(3H,t,J=7.2Hz), 2.56(3H,s), 4.28(2H,q,J=7.2Hz), 4.79(2H,s), 7.57(2H,d,J=8.8Hz), 8.30(2H,d,J=8.8Hz), 10.30(1H,s).
IR (KBr): 1748, 1719, 1663, 1522, 1460 cm⁻¹.

### Reference Example 8

### Production of 2,4(1H,3H)-dioxo-1-(2-fluorobenzyl)-6-(4-nitrophenyl)-5-methylthieno[2,3-d]pyrimidin-3-acetic acid ethyl ester:

To a solution of the compound produced in Reference Example 7 (700 mg, 1.80 mmol) in dimethylformamide (10 ml) were added potassium carbonate (372 mg, 2.70 mmol), potassium iodide (299 mg, 1.80 mmol) and 2-fluorobenzyl chloride (0.43 ml, 3.60 mmol). The mixture was stirred for 2 hours at room temperature. The reaction mixture was concentrated, and the concentrate was partitioned between ethyl acetate and an aqueous sodium chloride solution. The aqueous layer was extracted with ethyl acetate. The combined extract was washed with an aqueous sodium chloride solution, which was then dried (MgSO₄), followed by distilling off the solvent under reduced pressure. The residue was chromatographed on silica gel to give a white powder (500 mg, 56%).
m.p.155-158°C.

| Elemental Analysis for C₂₄H₂₀N₃O₆SF·0.5H₂O: | | | |
|---|---|---|---|
| | C(%) | H(%) | N(%) |
| Calcd. | 56.91 ; | 4.18 ; | 8.30 |
| Found | 56.74 ; | 3.84 ; | 8.25 |

¹H-NMR (200MHz, CDCl₃) δ: 1.32(3H,t,J=7.2Hz), 3.84(3H,s), 4.27(2H,q,J=7.2Hz), 4.84(2H,s), 5.30(2H,s), 7.06-7.33(4H,m), 7.54(2H,d,J=8.9Hz), 7.27(2H,d,J=8.9Hz).
IR (KBr): 1748, 1711, 1673, 1520, 1491 cm⁻¹.

### Reference Example 9

Starting from the compounds which are produced in Reference Example 5 and 6, compounds which are produced in accordance with the method described in Reference Example 8 are set forth in Table 3.

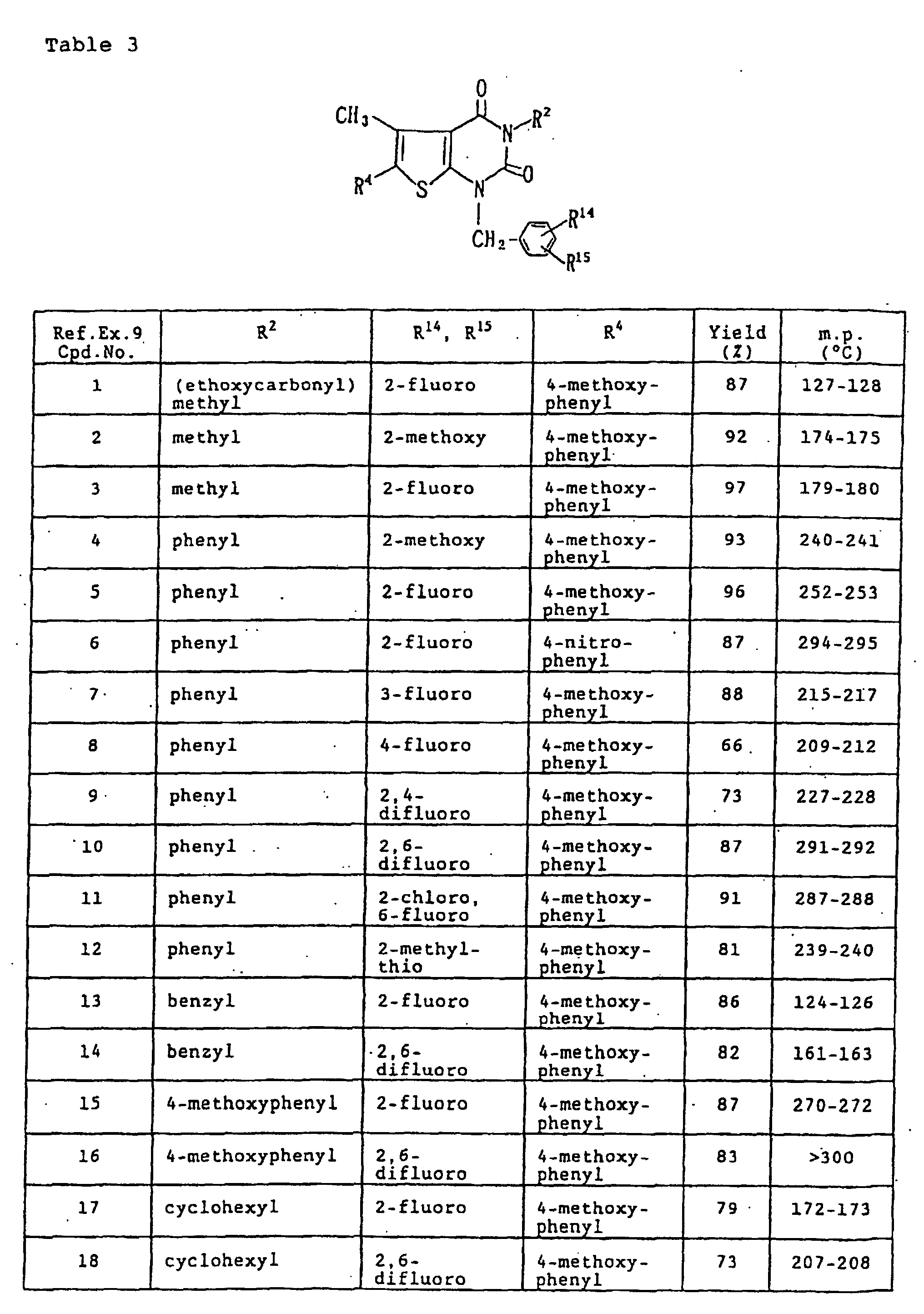

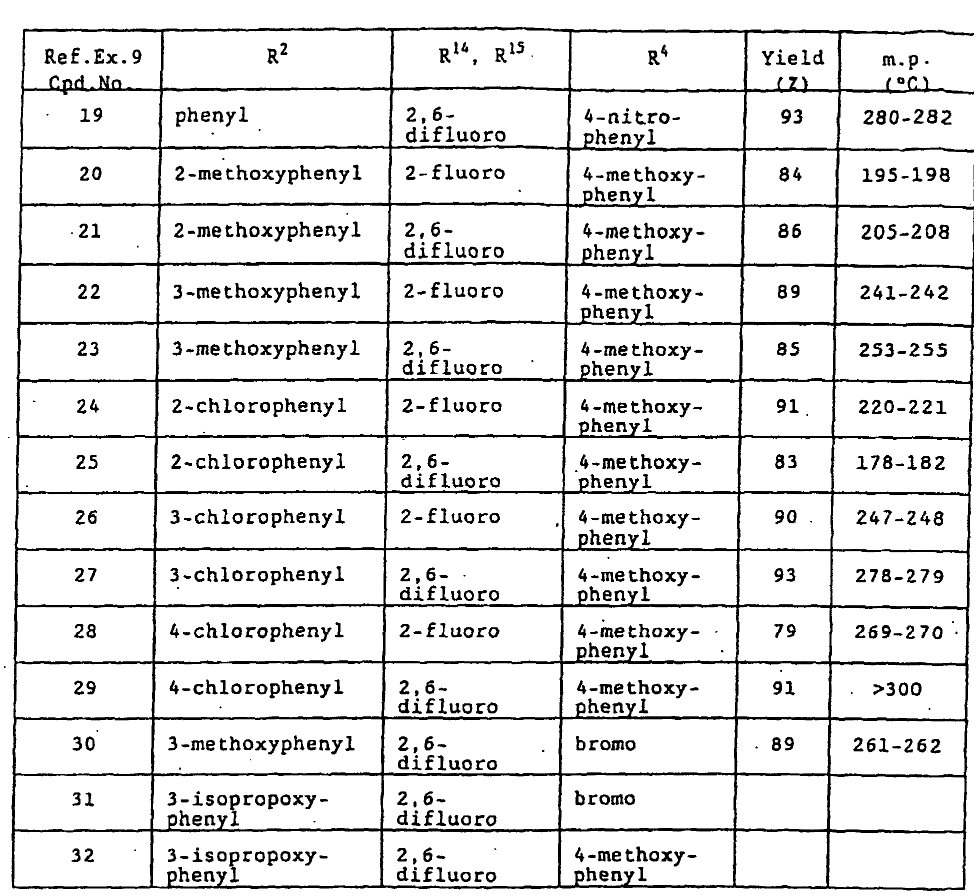

### Reference Example 10

### Production of 5-bromomethyl-2,4(1H,3H)-dioxo-1-(2-fluorobenzyl)-6-(4-nitrophenyl)thieno[2,3-d]pyrimidin-3-acetic acid ethyl ester:

A mixture of the compound produced in Reference Example 8 (0.300 g, 0.603 mmol), N-bromosuccinimide (0.107 g, 0.603 mmol), α,α'-azobisisobutyronitrile (10 mg, 0.60 mmol) and carbon tetrachloride (15 ml) was refluxed for 2 hours. Upon cooling resulting insolubles were filtered off from the reaction mixture. The filtrate was diluted with chloroform. The organic layer was washed with an aqueous sodium chloride solution and dried (MgSO₄), then the solvent was distilled off under reduced pressure. The residue was recrystallized from ethyl acetate to give colorless needles (0.284 g, 82%), m.p.165-167°C.

| Elemental Analysis for C₂₄H₁₉N₃O₆SBrF: | | | |
|---|---|---|---|
| | C(%) | H(%) | N(%) |
| Calcd. | 50.01 ; | 3.32 ; | 7.29 |
| Found | 49.87 ; | 3.27 ; | 7.23 |

¹H-NMR (200MHz, CDCl₃) δ: 1.31(3H,t,J=7.1Hz), 4.26(2H,q,J=7.1Hz), 4.78(2H,s), 4.86(2H,s), 5.30(2H,s), 7.07-7.37(4H,m), 7.75(2H,d,J=8.8Hz), 8.33(2H,d,J=8.8Hz).
IR (KBr): 1713, 1673, 1524, 1477 cm⁻¹.

### Reference Example 11

Starting from the compounds which is produced in Reference Example 9, compounds which are produced in accordance with the method described in Reference Example 10 are set forth in Table 4. The compounds 30 to 33 is produced from the compounds 30 or 31 of Reference Example 9 by the method of Example 18.

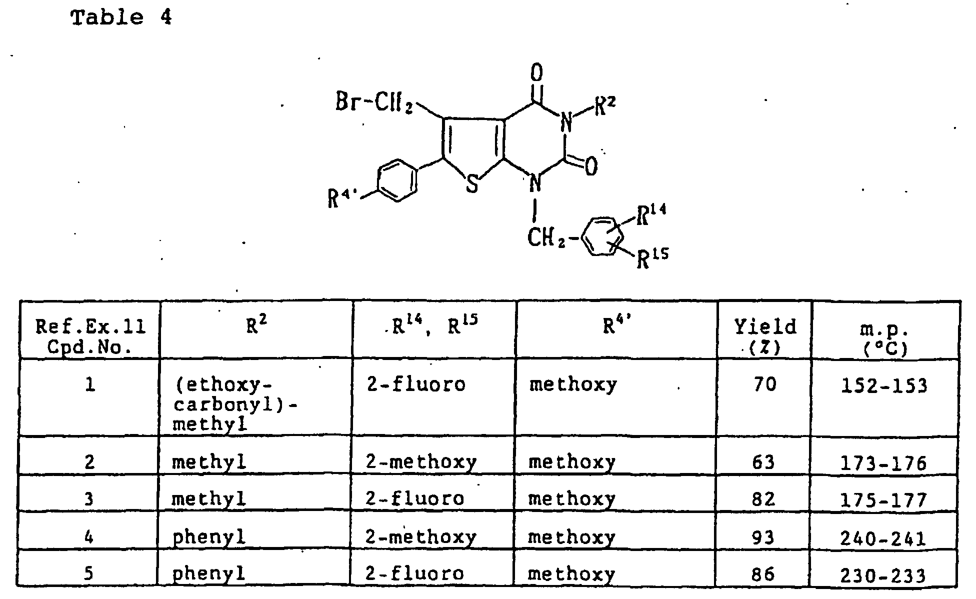

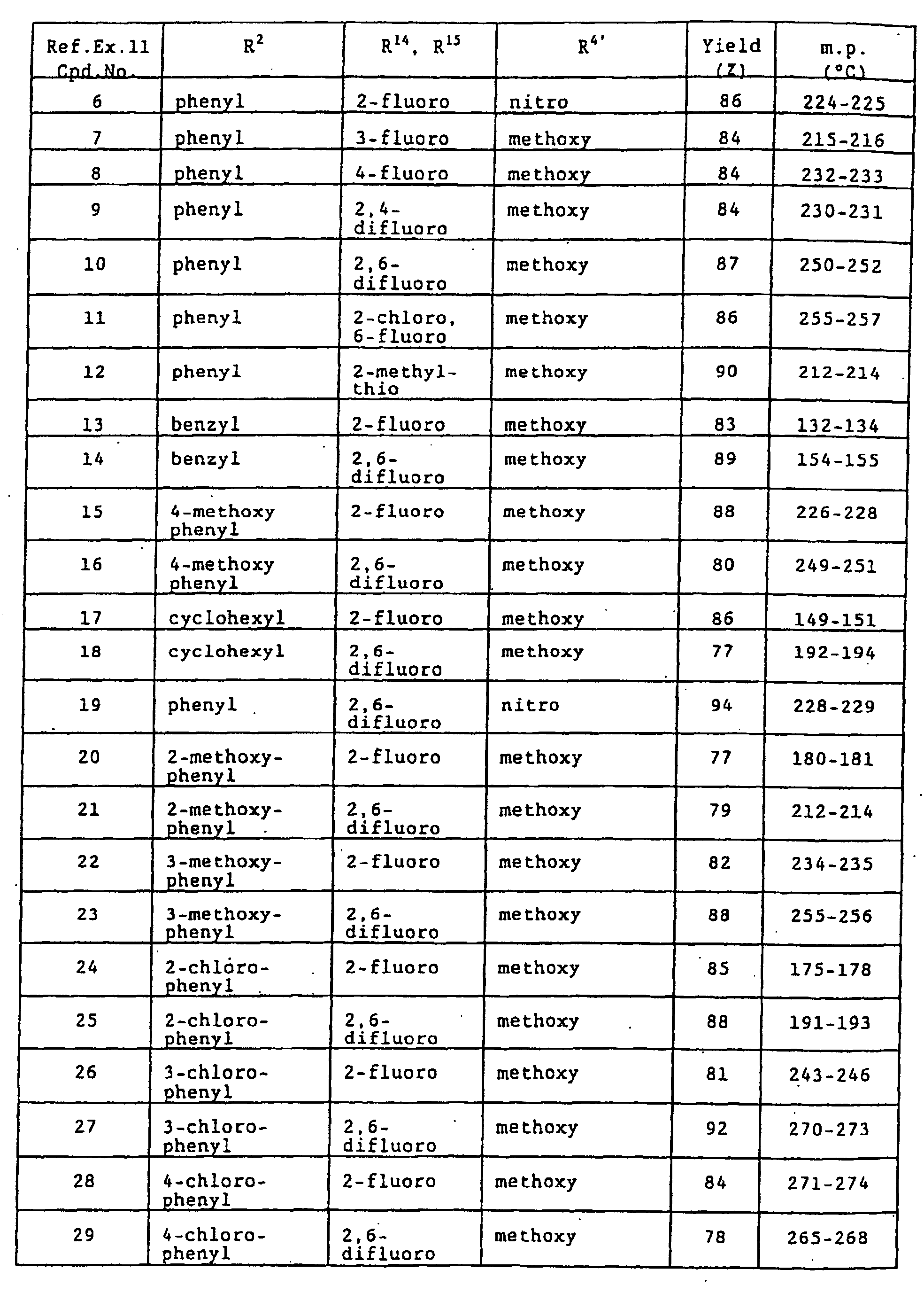

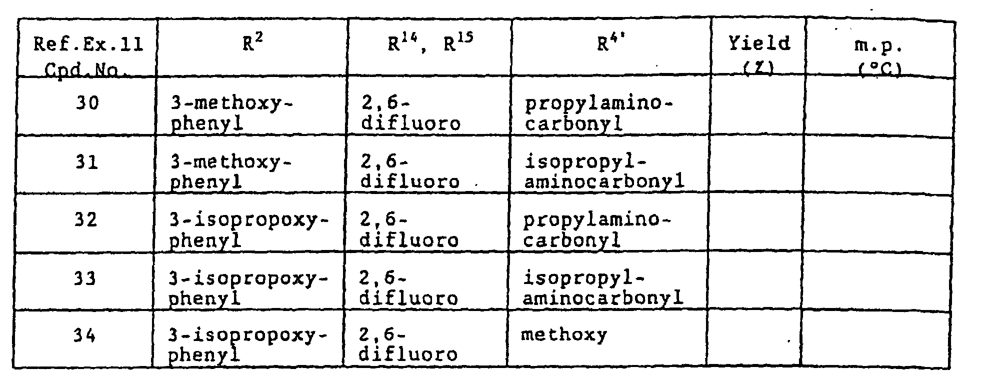

### Reference Example 12

### Production of 5-(N-benzyl-N-methylaminomethyl)-2,4(1H,3H)-dioxo-1-(2-fluorobenzyl)-6-(4-nitrophenyl)thieno[2,3-d]pyrimidin-3-acetic acid ethyl ester hydrochloride:

To a solution of the compound produced in Reference Example 10 (0.270 g, 0.47 mmol) in dimethylformamide (10 ml) were added, under ice-cooling, ethyl diisopropylamine (0.12 ml, 0.710 mmol) and benzylmethyl amine (0.07 ml, 0.56 mmol). The mixture was stirred for 20 hours at room temperature. The reaction mixture was concentrated, and the concentrate was partitioned between ethyl acetate and a saturated aqueous solution of sodium hydrogencarbonate. The aqueous layer was extracted with ethyl acetate. Organic layers were combined and dried (MgSO₄), then the solvent was distilled off under reduced pressure. The residue was chromatographed on silica gel to give a colorless oil (0.297 g, 100%). To a solution of this oil in ethyl acetate was added, under ice-cooling, 1N solution of hydrogen chloride in ether. The mixture was stirred for 10 minutes at the same temperature. The reaction mixture was concentrated under reduced pressure, and the concentrate was crystallized from ethyl acetate - ether to give the corresponding hydrochloride (0.084 g) as white crystals.
m.p. 120-128°C

| Elemental Analysis for C₃₂H₂₉N₄O₆SF·HCl·H₂O: | | | |
|---|---|---|---|
| | C(%) | H(%) | N(%) |
| Calcd. | 57.27 ; | 4.81 ; | 8.35 |
| Found | 57.23 ; | 4.55 ; | 8.42 |

IR (KBr): 1711, 1665, 1522, 1493 cm⁻¹.

### Reference Example 13

### Production of 3-isobutyl-2,4(1H,3H)-dioxo-5-methyl-6-(4-methoxyphenyl)thieno[2,3-d]pyrimidine:

A mixture of isovaleric acid (1.15 ml, 10.03 mmol), diphenylphosphoryl azide (2.83 g, 10.30 mmol), triethylamine (1.45 ml, 10.03 mmol) and benzene (15 ml) was heated for one and half hour under reflux, to emerge isobutyl isocyanate. To the resultant mixture, the compound produced in Reference Example 2 (2.00 g, 6.85 mmol) and benzene (5 ml) were added, and the mixture was heated under reflux for 4 days. The reaction mixture was subjected to distribution procedure with-ethyl acetate and an aqueous sodium chloride solution. The water layer was extracted with ethyl acetate, and the combined extracts were washed with an aqueous sodium chloride solution and dried with MgSO₄, and the solvent was removed under reduced . pressure. The residue was chromatographed on silica gel to obtain white powders (2.64 g, 99%). The obtained urea derivative was dissolved in ethanol (30 ml), 28% sodium methoxide (3.93 g, 20.37 mmol) was added to the solution, the mixture was stirred at room temperature for 16 hours, 1N hydrochloric acid (22 ml, 22 mmol) was added. The solvent, ethanol, was distilled off under reduced pressure. The resulting residue was filtrated, washed with water-ethanol, dried under reduced pressure, and then crystallized from ethanol, to give white needles (.1.61 g, 70%).
m.p. 215-216°C.

| Elemental Analysis for C₁₈H₂₀N₂O₃S: | | | |
|---|---|---|---|
| | C(%) | H(%) | N(%) |
| Calcd. | 62.77 ; | 5.85 ; | 8.13 |
| Found | 62.75 ; | 5.82 ; | 8.04. |

¹H-NMR (300MHz, CDCl₃) δ: 0.96(6H,d,J=6.8Hz), 2.20(1H,sept,J=6.8Hz), 2.50(3H,s), 3.85-3.87(5H,m), 6.96(2H,d,J=8.8Hz), 7.33(2H,d,J=8.8Hz), 9.50(1H,s).
IR (KBr):1711, 1657, 1537, 1499, 1458 cm⁻¹.

### Reference Example 14

Employing the compounds which are produced in Reference Example 2 or 4 as a starting material, compounds which are produced in accordance with the method described in Reference Example 13 are set forth in Table 5.

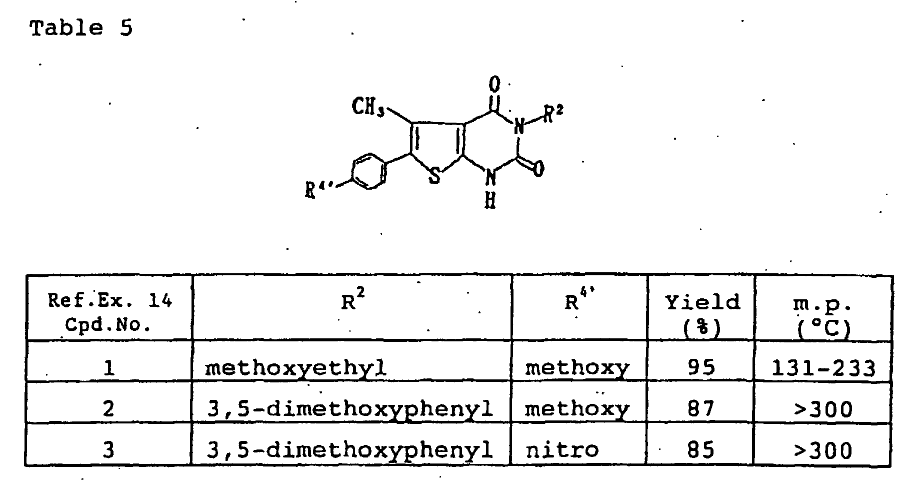

### Reference Example 15

### Production of 2-amino-4-methyl-5-(4-methoxyphenyl)thiophene-3-carboxylic acid:

To an ethanol solution (60 ml) of the compound (3.0 g, 10.3 mmol) produced in Reference Example 2, 2N sodium hydroxide (20.0 ml, 40.0 mmol) was added and the mixture was heated under reflux for 1.5 hours. After cooling, 2N hydrochloric acid (20.0 ml, 40.0 mmol) was added to the reaction mixture to neutralize the solution, and the solution was extracted with ethyl acetate. The organic layer was washed with an aqueous sodium chloride solution, and then dried with MgSO₄. The solvent was distilled off under reduced pressure, and the residue was washed with ether-hexane to give pale yellowish, powder (2.2 g, 91%).
m.p. 142-145°C.
¹H-NMR (200MHz, DMSO-d₆) δ: 2.22(3H,s), 3.79(3H,s), 6.98(2H,d,J=8.8Hz), 7.25(2H,d,J=8.8Hz), 7.39(2H,s).
IR (KBr): 3470, 1647, 1576, 1508, 1475 cm⁻¹.

### Reference Example 16

### Production of 2,4(1H)-dioxo-6-(4-methoxyphenyl)-5-methylthieno[2,3-d]oxazine:

To a dioxane solution (10 ml) of the compound (6.00 g, 22.8 mmol) produced in Reference Example 15, triphosgene (6.76 g, 22.8 mmol) was added, and the mixture was stirred at 100°C for 4 hours. After the reaction, the reaction solution was concentrated, then the residue was filtered and washed with ether to give pale yellowish powder (596 g, 90%) was obtained.
m.p. 209-210°C.
¹H-NMR (200MHz, DMSO-d₆) δ: 2.36(3H,s), 3.82(3H,s), 7.06(2H,d,J=8.8Hz), 7.41(2H,d,J=8.8Hz), 10.50(1H,s).
IR (KBr): 1779, 1709, 1533, 1497 cm⁻¹.

### Reference Example 17

### Production of 2,4(1H)-dioxo-1-(2-fluorobenzyl)-6-(4-methoxyphenyl)-5-methylthieno[2,3-d]oxazine:

To a.dimethylformamide (30 ml) solution of the compound (4.80 g, 16.59 mmol) produced in Reference Example 16, potassium carbonate (3.43 g, 24.88 mmol), potassium iodide (2.75 g, 16.59 mmol) and 2-fluorobenzylchloride (2.96 ml, 24.88 mmol) were added, and the mixture was stirred at room temperature for 2 hours. The reaction mixture was concentrated, the residue was subjected to distribution with ethyl acetate and an aqueous sodium chloride solution. The aqueous layer was extracted with ethyl acetate, the extracts were combined and washed with an aqueous sodium chloride solution and dried with MgSO₄, and the solvent was distilled off under reduced pressure. The obtained residue was subjected to purification by silica gel column chromatography to give white crystals (4.87 g, 74%).
m.p. 162-163°C.
¹H-NMR (200MHz, CDCl₃) δ: 2.43(3H,s), 3.84(3H,s), 5.21(2H,s), 6.95(2H,d,J=8.8Hz), 7.05-7.44(6H,m).
IR (KBr): 1769, 1719, 1562, 1531, 1493 cm⁻¹.
FAB-MS m/z: 398.1(MH⁺).

### Reference Example 18

### Production of 2,4(1H)-dioxo-1-(2,6-difluorobenzyl)-6-(4-methoxyphenyl)-5-methylthieno[2,3-d]oxazine:

In substantially the same procedure as described in Reference Example 17, using 2,6-difluorobenzylchloride (1.18 g, 7.26 mmol) in place of 2-fluorobenzylchloride, from the compound (2.00 g, 6.91 mmol) obtained in Reference Example 16, potassium carbonate (0.95 g, 6.91 mmol) and potassium iodide (1.15 g, 6.91 mmol), the titled compound was produced as colorless crystals (2.34 g, 82%).
m.p. 189-190°C (recrystallized from ethyl acetate-hexane).
¹H-NMR (300MHz, CDCl₃) δ: 2.42(3H,s), 3.84(3H,s), 5.27(2H,s), 6.90-6.96(4H,m), 7.24-7.36(3H,m).
IR (KBr): 1775, 1731, 1528, 1468 cm⁻¹.

### Reference Example 19

### Production of 2,4-(1H,3H)-dioxo-1-(2-fluorobenzyl)-6-(4-methoxyphenyl)-3-(3-methoxypropyl)-5-methylthieno[2,3-d]pyrimidine:

To a dichloromethane (12 ml) solution of the compound obtained in Reference Example 17, 3-methoxypropylamine (0.17 ml, 1.67 mmol) was added under ice-cooling, and the mixture was stirred at room temperature for 1 hour. The residue obtained by concentrating the reaction mixture was subjected to distribution with dichloromethane and an aqueous sodium chloride solution. The aqueous layer was extracted with dichloromethane, the extracts were combined, the extracts was washed with an aqueous sodium chloride solution and dried with MgSO₄, and then the solvent was distilled off. Thus obtained residue was purified by silica gel column chromatography to give a white powder (524 mg, 78%). The obtained amine derivative was dissolved in tetrahydrofuran (20 ml), and to this solution triphosgene (351 mg, 1.18 mmol) and triethylamine (0.15 ml, 2.37 mmol) was added, and the mixture was stirred for 1.5 hours under heating. After cooling, the reaction mixture was extracted with ethyl acetate, the organic layer was washed with an aqueous sodium chloride solution and dried with MgSO₄, and the solvent was distilled off under reduced pressure. Thus obtained residue was purified by silica gel chromatography, and after drying it was subjected to recrystallization with ethyl acetate-hexane to give a white crystalline plate (398 mg, 72%).
m.p. 113-115°C.

| Elemental Analysis for C₂₅H₂₅N₂O₄SF: | | | |
|---|---|---|---|
| | C(%) | H(%) | N(%) |
| Calcd. | 64.09 ; | 5.38 ; | 5.98 |
| Found | 63.89 ; | 5.39 ; | 5.92. |

¹H-NMR (300MHz, CDCl₃) δ: 2.00(2H,quint,J=6.7Hz), 2.50(3H,s), 3.34(3H,s), 3.50(2H,t,J=6.7Hz), 3.83(3H,s), 4.18(2H,t,J=6.7Hz), 5.26(2H,s), 6.93(2H,d,J=8.8Hz), 7.07-7.12(2H,m), 7.24-7.29(4H,m).
IR (KBr): 1700, 1659, 1473 cm⁻¹.

### Reference Example 20

Employing the compounds which are produced in Reference Example 18 as a starting material, compounds which are produced in accordance with the method described in Reference Example 19 are set forth in Table 6.

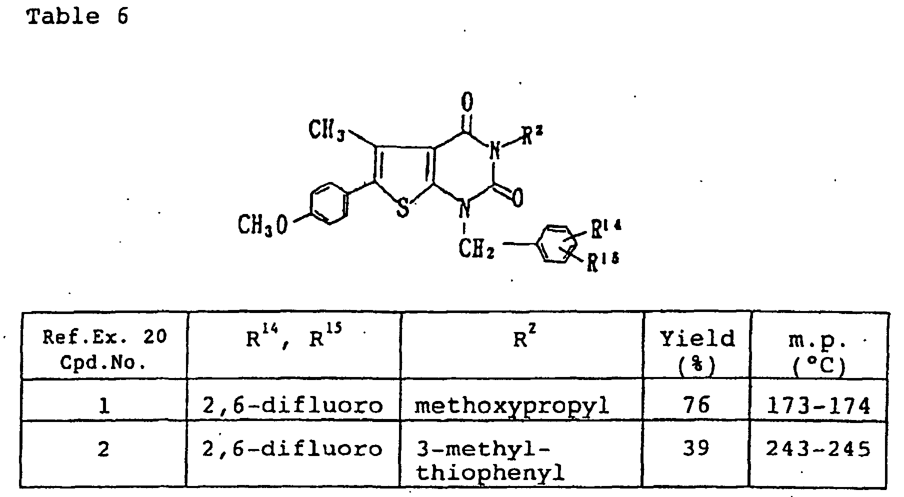

### Reference Example 21

### Production of 2,4(1H,3H)-dioxo-3-phenyl-5-methyl-6-(4-nitrophenyl)thieno[2,3-d]pyrimidine:

To a pyridine (30 ml) solution of the compound (5.00 g, 16.32 mmol) obtained in Reference Example 4, phenylisocyanate (2.66 ml, 24.48 mmol) was added. The mixture was stirred at 45°C for 6 hours, the reaction mixture was concentrated under reduced pressure to give a residue. The residue was dissolved in ethanol (6 ml). To the solution was added 28% sodium methoxide (7.86 g, 40.80 mmol), the mixture was stirred at room temperature for 2 hours, to the resultant was added 2N hydrochloric acid (25 ml, 50 mmol), and the solvent, ethanol, was distilled off under reduced pressure. Thus obtained residue was subjected to filtration, washed with water-ethanol, dried under reduced pressure, and recrystallized by ethanol to give yellow power (6.09 g, 98%).
m.p. >300°C.

| Elemental Analysis for C₁₉H₁₃N₃O₄S·0.3H₂O: | | | |
|---|---|---|---|
| | C(%) | H(%) | N(%) |
| Calcd. | 59.30 ; | 3.56 ; | 10.92 |
| Found | 59.56 ; | 3.52 ; | 10.93. |

¹H-NMR (300MHz, DMSO-d₆) 6: 2.50(3H,s), 7.31-7.46(5H,m), 7.78(2H,d,J=8.8Hz), 8.32(2H,d,J=8.8Hz), 12.50(1H,s).
IR (KBr): 1715, 1657, 1593, 1510 cm⁻¹.

### Reference Example 22

### Production of 2,4(1H,3H)-dioxo-5-methyl-3-(3-methoxyphenyl)-6-(4-nitrophenyl)thieno[2,3-d]pyrimidine:

In substantially the same procedure as described in Reference Example 21, using 3-methoxyphenylisocyanate (1.57 ml, 12.0 mmol) in place of phenylisocyanate, from the compound (3.06 g, 10.00 mmol) obtained in Reference Example 4 and 28% sodium methoxide (4.82 g, 25.00 mmol), the titled compound was produced as colorless crystals (3.15 g, 77%).
m.p. >300°C.

| Elemental Analysis for C₂₀H₁₅N₃O₅S: | | | |
|---|---|---|---|
| | C(%) | H(%) | N(%) |
| Calcd. | 58.67 ; | 3.69 ; | 10.26 |
| Found | 58.76 ; | 3.67 ; | 10.32. |

¹H-NMR (300MHz, CDCl₃) δ: 2.50(3H,s), 3.78(3H,s), 6.87(1H,d,J=8.1Hz), 6.92(1H,s), 7.00(1H,d,J=8.1Hz), 7.38(1H,t,J=8.1Hz), 7.77(1H,d,J=8.7Hz), 8.31(2H,d,J=8.7Hz), 12.48(1H,s).
IR (KBr): 1717, 1661, 1593, 1510, 1429 cm⁻¹.

### Reference Example 23

### Production of 2,4(1H,3H)-dioxo-1-(2,6-difluorobenzyl)-5-methyl-3-(3-methylsulfinylphenyl)-6-(4-methoxyphenyl)thieno[2,3-d]pyrimidine:

To a dichloromethane (20 ml) solution of the compound 2 (200 mg, 0.37 mmol) obtained in Reference Example 20 (Table 6), m-chloroperbenzoic acid (129 mg, 0.37 mmol) was added under ice-cooling. The mixture was stirred for 30 minutes, and the reaction mixture was subjected to distribution with dichloromethane and an aqueous sodium chloride solution. The aqueous layer was extracted with dichloromethane, the combined extracts were dried with an aqueous sodium chloride solution and dried with MgSO₄, and the solvent was distilled off under reduced pressure. Thus obtained residue was purified by silica gel column chromatography to give white powders (183 mg, 89%).
m.p. 267-268°C.
¹H-NMR (300MHz, CDCl₃) δ: 2.46(3H,s), 2.79(3H,s), 3.85(3H,s), 5.35(2H,s), 6.90-6.97(4H,m), 7.33-7.72(7H,m).
IR (KBr): 1717, 1667, 1628, 1562, 1533 cm⁻¹.
FAB-MS m/z: 553.1(MH⁺).

### Reference Example 24

### Production of 2,4(1H,3H)-dioxo-1-(2,6-difluorobenzyl)-5-methyl-3-(3-methylsulfonylphenyl)-6-(4-methoxyphenyl)thieno[2,3-d]pyrimidine:

In substantially the same procedures as described in Reference Example 23, using m-chloroperbenzoic acid (62 mg, 0.18 mmol) again, from the compound (100 mg, 0.18 mmol) obtained in Reference Example 23, the titled compound was produced as colorless crystals (98 mg, 95%).
m.p. 256-257°C.
¹H-NMR (300MHz, CDCl₃) δ: 2.46(3H,s), 3.10(3H,s), 3.85(3H,s), 5.36(2H,s), 6.90-6.97(4H,m), 7.29-8.01(7H,m).
IR (KBr): 1719, 1665, 1531, 1473 cm⁻¹.
FAB-MS m/z: 569.1(MH⁺).

### Reference Example 25

Employing the compounds which are produced in accordance with the methods of Reference Example 13, 14, 21 or 22 as a starting material, compounds which are produced in accordance with the method described in Reference Example 17 are set forth in Table 7.

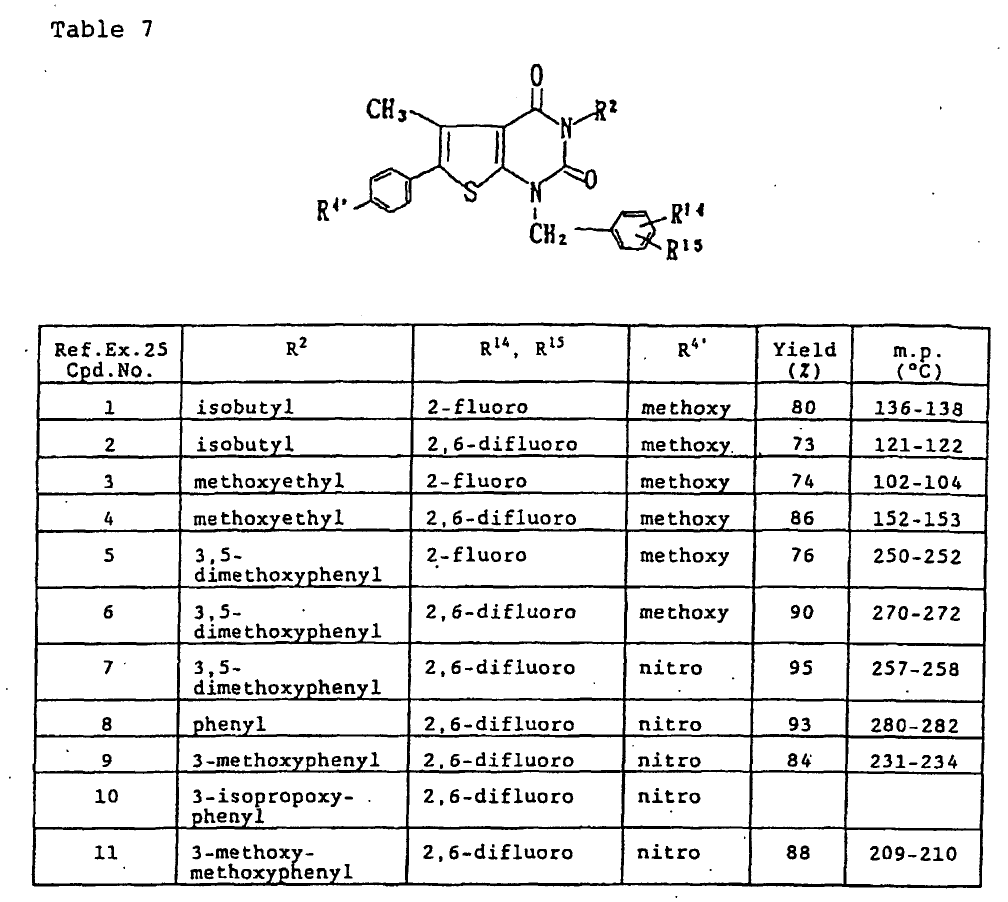

### Reference Example 26

### Production of 2,4(1H,3H)-dioxo-1-(2-fluorobenzyl)-5-bromomethyl-6-(4-methoxyphenyl)-3-(3-methoxypropyl)thieno[2,3-d]pyrimidine:

A mixture of the compound (270 mg, 0.576 mmol) obtained in Reference Example 19, N-bromosuccinimide (103 mg, 0.576 mmol), α,α'-azobisisobutylonitrile 10 mg, 0.058 mmol) and carbon tetrachloride (10 ml) was heated under reflux. After cooling, insolubles were removed by filtration, the filtrate was diluted with chloroform. The organic layer was washed with an' aqueous sodium chloride solution and dried with MgSO₄, and then the solvent was distilled off under reduced pressure. Thus obtained residue was recrystallized by ethyl acetate to give colorless powders (294 mg, 93%).
m.p. 105-107°C.
¹H-NMR (300MHz, CDCl₃) δ: 2.01(2H,quint,J=6.7Hz), 3.33(3H,s), 3.50(2H,t,J=6.7Hz), 3.85(3H,s), 4.21(2H,t,J=6.7Hz), 4.81(2H,s), 5.27(2H,s), 6.98(2H,d,J=8.8Hz), 7.09-7.34(4H,m), 7.49(2H,d,J=8.8Hz).
IR (KBr): 1713, 1661, 1628, 1541 cm⁻¹.
FAB-MS m/z: 548.1(MH⁺).

### Reference Example 27

Employing the compounds which are produced in Reference Examples 20, 23, 24 or 25 as starting materials, compounds which are produced in accordance with the method described in Reference Example 26 are set forth in Table 8.

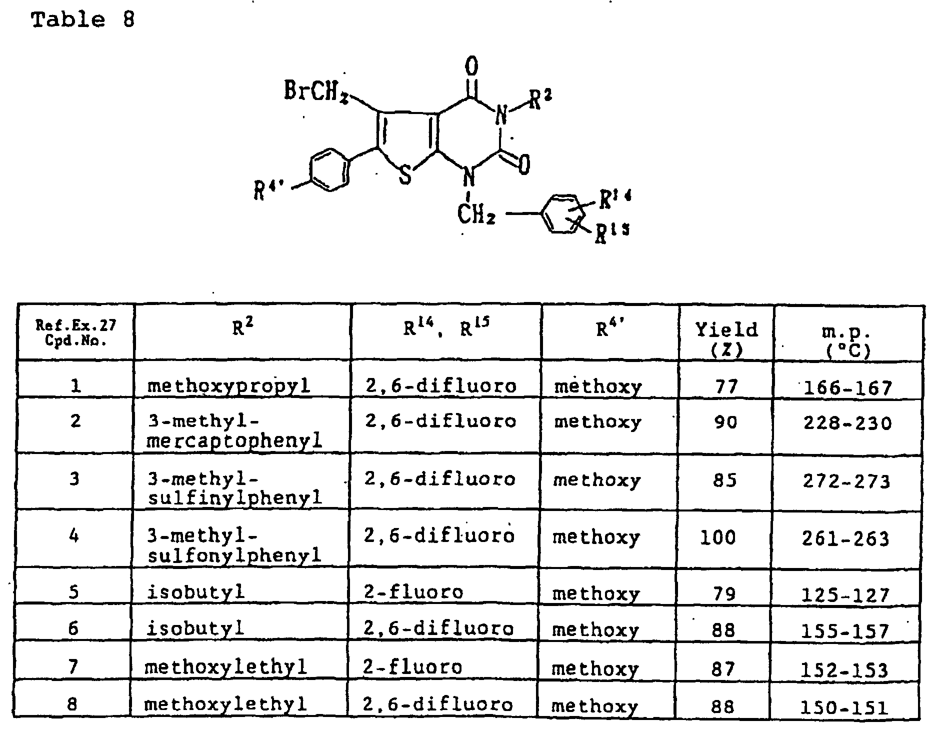

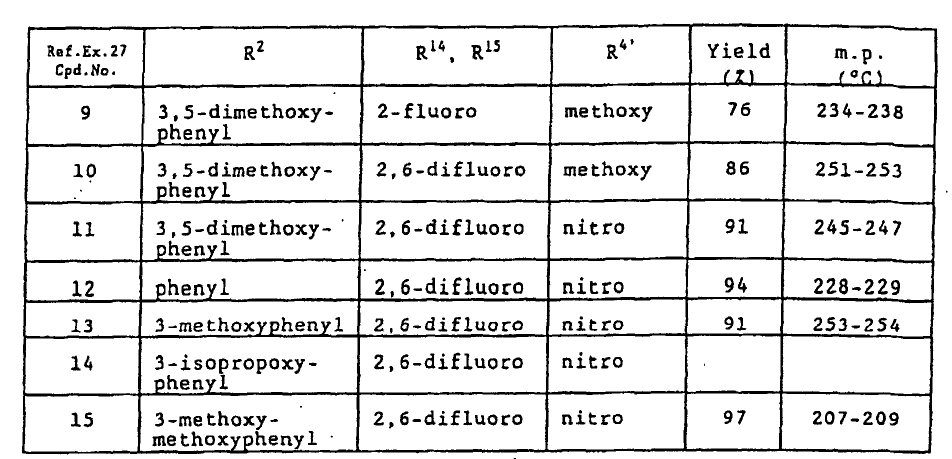

### Example 1

### Production of 2,4(1H,3H)-dioxo-6-(4-methoxyphenyl)-3-phenyl-1-(2-fluorobenzyl)-5-(N-benzyl-N-methylaminomethyl)thieno[2,3-d]pyrimidine hydrochloride:

To a solution of the compound 5 produced in Reference Example 11 (0.150 g, 0.310 mmol) in dimethylformamide (10 ml), with ice-cooling, were added ethyldiisopropylamine (0.08 ml, 0.460 mmol) and methylbenzylamine (0.05 ml, 0.370 mmol). After stirring for 2 hours at room temperature, the reaction mixture was concentrated. The residue was partitioned between ethyl acetate and a saturated aqueous solution of sodium bicarbonate. The aqueous layer was extracted with ethyl acetate. The combined organic layer was dried (MgSO₄). The solvent was distilled off under reduced pressure, and the residue was chromatographed on silica gel to give a colourless oil (0.159 g, 97%). To the solution of this oil in ethyl acetate (4 ml) was added, with ice-cooling, an 1N solution of hydrogen chloride in ether (0.3 ml). After stirring for 10 minutes under ice-cooling, the reaction mixture was concentrated with reduced pressure. The residue was crystallized from ethyl acetate-ether to give a titled hydrochloride (0.144 g) as white crystals.
m.p. 140-143°C

| Elemental Analysis for C₃₅H₃₀N₃O₃SF·HCl·H₂O: | | | |
|---|---|---|---|
| | C(%) | H(%) | N(%) |
| Calcd. | 65.05 ; | 5.14 ; | 6.50 |
| Found | 65.14 ; | 5.03 ; | 6.37 |

IR(KBr) 1711, 1665, 1543, 1477 cm⁻¹.

### Example 2

Starting from the compounds which are produced in Reference Example 11, compounds which are produced in accordance with the method described in Example 1 are set forth in Table 9.

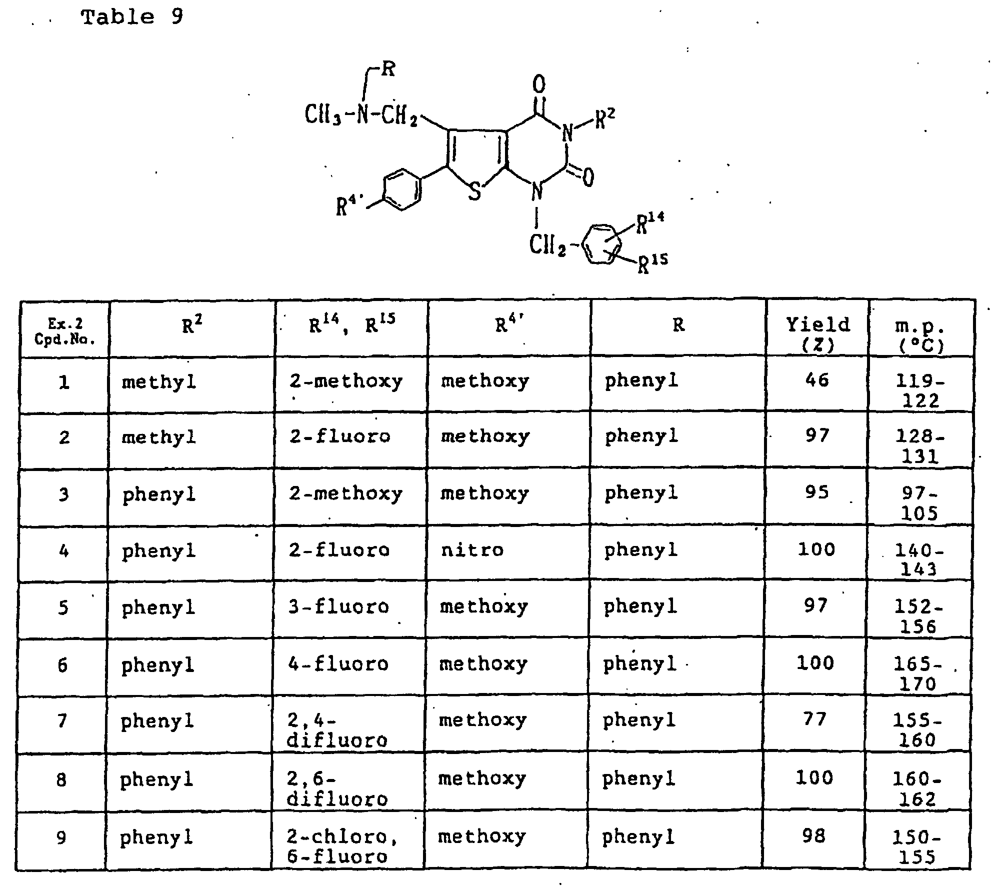

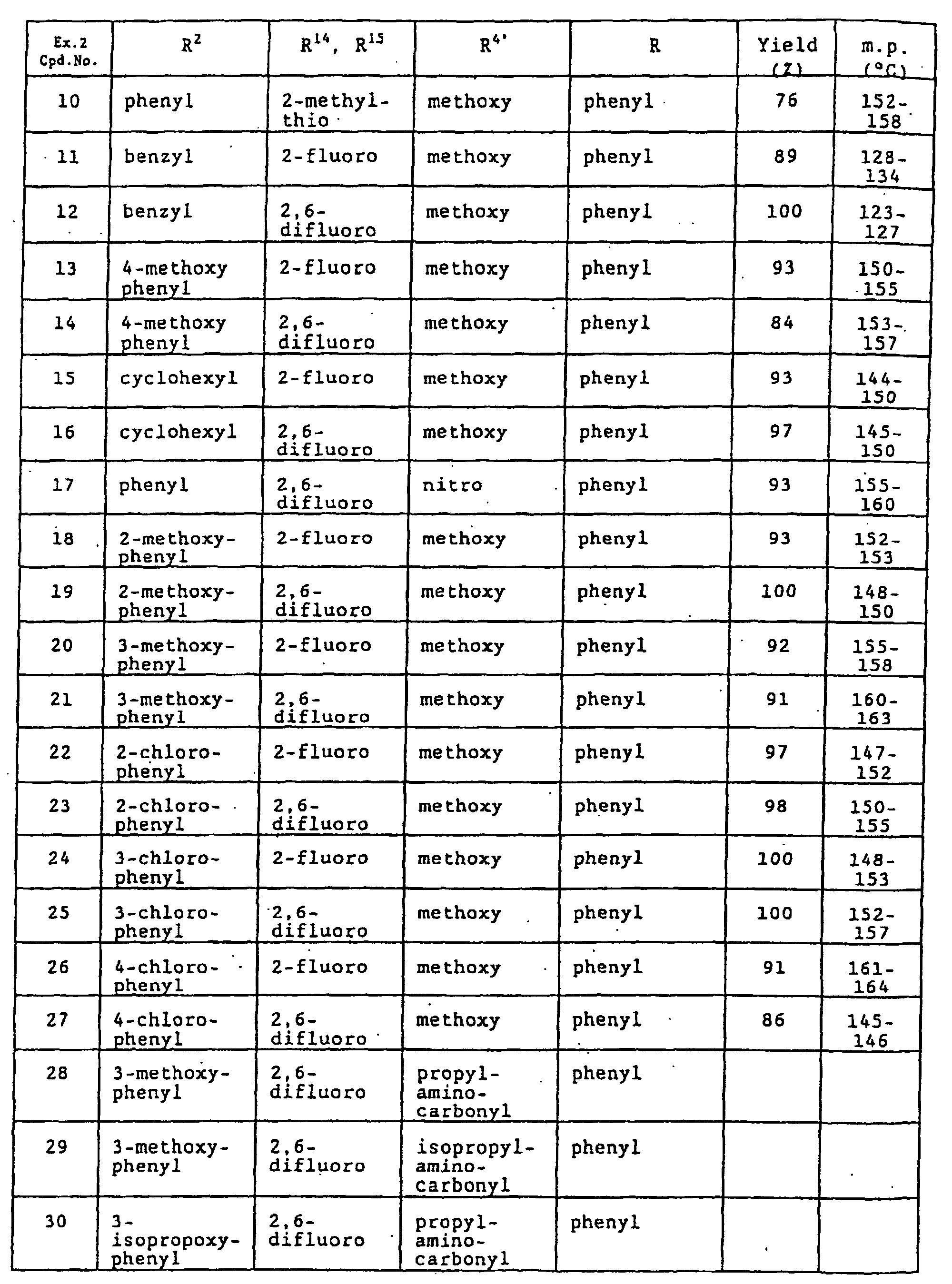

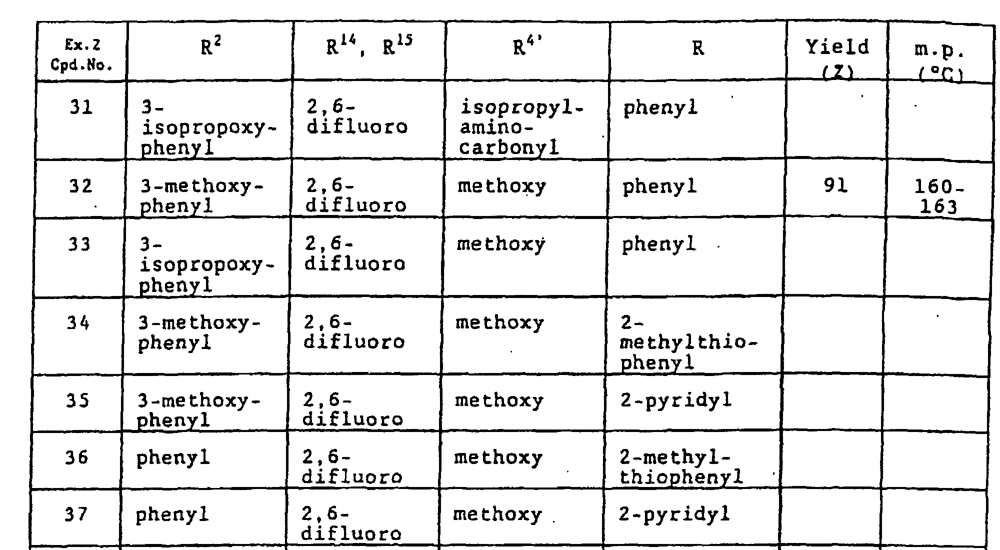

### Example 3

### Production of 6-(4-aminophenyl)-2,4(1H,3H)-dioxo-1-(2-fluorobenzyl)-3-phenyl-5-(N-methyl-N-benzylaminomethyl)thieno[2,3-d]pyrimidine:

The compound 4 produced in Example 2 (0.15 g, 0.247 mmol) was dissolved in ethanol (15 ml), to which was added 10% palladium-carbon (15 mg). The mixture was hydrogenized for 8 hours at room temperature under atmospheric pressure in an atmosphere of hydrogen. The reaction mixture was filtrated with celite, and the filtrate was concentrated under reduced pressure. The concentrate was chromatographed on silica gel to give a yellow crystalline amorphous (0.046 g, 32%).
¹H-NMR (300MHz, CDCl₃) δ: 2.05(3H,s), 3.57(2H,s), 3.81(2H,br s), 3.89(2H,s), 5.29(2H,s), 6.69(2H,d,J=8.7Hz), 7.05-7.56(16H,m).
FAB-Mass m/z 577(MH)⁺

### Example 4

### Production of 6-(aminophenyl)-2,4-(1H,3H)-dioxo-1-(2,6-difluorobenzyl)-5-(N-methyl-N-benzylaminomethyl)-3-phenylthieno[2,3-d]pyrimidine:

Starting from the compound No. 17 produced in Example 2, the titled compound as crystalline amorphous (65%) was produced in accordance with the method described in Example 3.
¹H-NMR (300MHz, CDCl₃) δ: 2.05(3H,s), 3.56(2H,s), 3.81(2H,br s), 3.88(2H,s), 5.36(2H,s), 6.71(2H,d,J=8.7Hz), 6.91(2H,t,J=8.7Hz), 7.21-7.53(13H,m).

### Example 5

### Production of 6-(4-acetylaminophenyl)-2,4(1H,3H)-dioxo-1-(2-fluorobenzyl)-5-(N-methyl-N-benzylaminomethyl)-3-phenylthieno[2,3-d]pyrimidine:

The compound produced in Example 3 (0.63 g, 0.11 mmol) was dissolved in anhydrous pyridine (5 ml), to which was added acetic anhydride (0.01 ml, 0.11 mmol). The mixture was stirred for 2 hours at room temperature. The reaction mixture was concentrated under reduced pressure. The concentrate was partitioned between methylene chloride (30 ml) and a saturated aqueous sodium chloride solution (10 ml).
The aqueous layer was again extracted with methylene chloride (30 ml). The combined organic layer was dried over magnesium sulfate, which was concentrated under reduced pressure. The concentrate was chromatographed on silica gel to give a colorless solid (0.01 g, 15%).
¹H-NMR (300MHz, CDCl₃) δ: 2.06(3H,s), 2.19(3H,s), 3.57(2H,s), 3.90(2H,s), 5.30(2H,s), 7.04-7.57(16H,s), 7.70(2H,d,J=8.4Hz).

### Example 6

Employing the compound produced in Example 3, as the starting material, in accordance with substantially the same procedure as described in Example 5, the following compounds were produced.
No. 1: 2,4(2H,3H)-Dioxo-1-(2-fluorobenzyl)-5-(N-methyl-N-benzylaminomethyl)-3-phenyl-6-(4-propionylaminophenyl)thieno[2,3-d]pyrimidine hydrochloride (yield: 86%, m.p. 172-175°C)
No. 3: 2,4(2H,3H)-Dioxo-1-(2-fluorobenzyl)-6-(4-methoxyacetylaminophenyl)-5-(N-methyl-N-benzylaminomethyl)-3-phenylthieno[2,3-d]pyrimidine hydrochloride (yield: 88%, m.p. 157-162°C)

### Example 7

### Production of 2,4(1H,3H)-dioxo-1-(2-fluorobenzyl)-5-(N-benzyl-N-methylaminomethyl)-6-(4-methoxyphenyl)-3-(3-methoxypropyl)thieno[2,3-d]pyrimidine:

To a dimethylformamide (10 ml) solution of the compound (284 mg, 0.519 mmol) obtained in Reference Example 26 were added ethyldiisopropylamine (0.140 ml, 0.780 mmol) and methylbenzylamine (0.080 ml, 0.620 mmol). The mixture was stirred at room temperature for 2 hours, the reaction mixture was concentrated, and the obtained residue was subjected to distribution with ethyl acetate and saturated sodium bicarbonate. The aqueous layer was extracted with ethyl acetate. The extract and the organic layer were combined, dried with MgSO₄, and the solvent was distilled off under reduced pressure. Thus obtained residue was purified by silica gel column chromatography to give colorless oily substance (280 mg, 92%). The oily substance was dissolved in ethyl acetate (4 ml), and to the solution iN solution of hydrogen chloride in ether (0.3 ml) under ice-cooling. The mixture was stirred under ice-cooling, and the reaction mixture was concentrated under reduced pressure. The residue was subjected to crystallization with ethyl acetate-ether to give a hydrochloride of the titled compound (220 mg) was obtained as white crystals.
m.p. 95-100°C.

| Elemental Analysis for C₃₅H₃₄N₃O₄SF·1.0HCl·0.5H₂O: | | | |
|---|---|---|---|
| | C(%) | H(%) | N(%) |
| Calcd. | 62.60 ; | 5.73 ; | 6.64 |
| Found | 62.73 ; | 5.67 ; | 6.56. |

IR (KBr): 1702, 1657, 1562, 1543, 1489 cm⁻¹.

### Example 8

Starting from the compounds which are produced in Reference Example 27, compounds which are produced in accordance with the method described in Example 7 are set forth in Table 10. The compound 19 and 20 are produced by hydrolyzing the compound 21 to produce the compound 22, and by reacting the compound 22 with alkyl halide in the presence of a base.

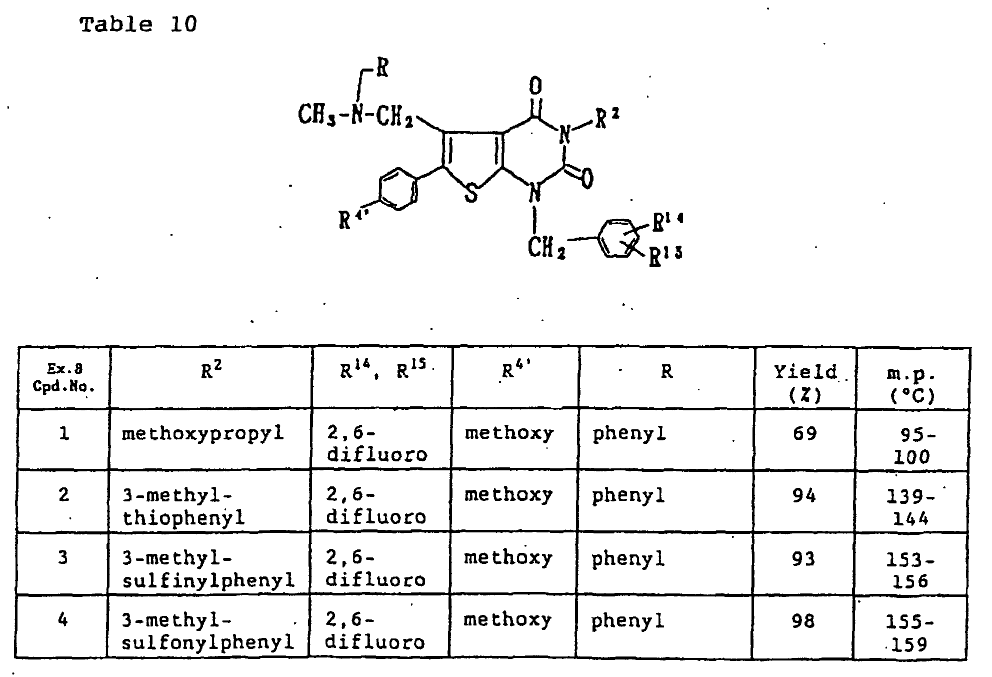

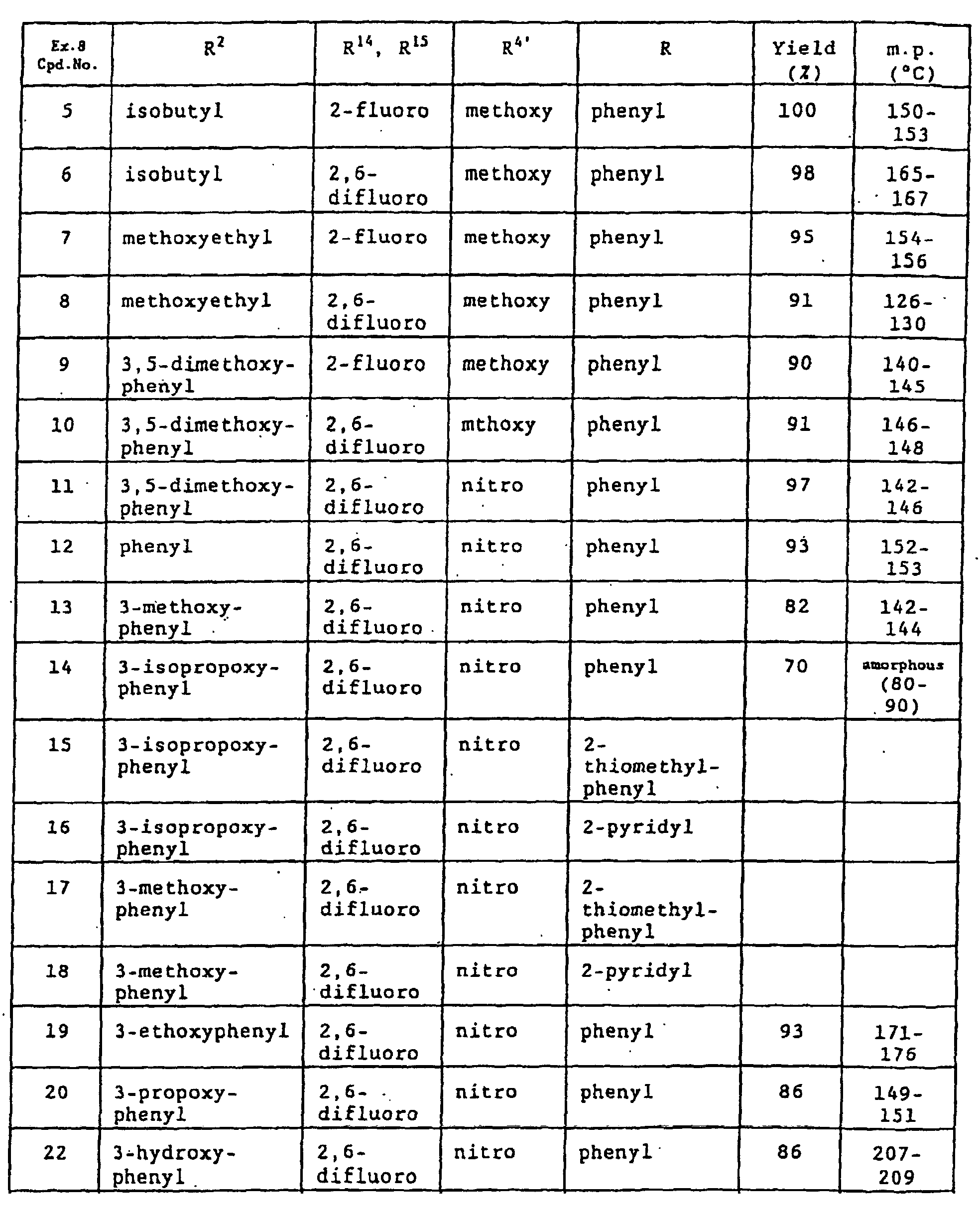

### Example 9

### Production of 6-(4-aminophenyl)-2,4(1H,3H)-dioxo-1-(2,6-difluorobenzyl)-5-(N-benzyl-N-methylaminomethyl)-3-(3-methoxyphenyl)thieno[2,3-d]pyrimidine hydrochloride:

To a formic acid (200 ml) solution of the compound 13 produced in Example 8, 50% paradium-carbon powder (0.90 g) was added under ice-cooling, and the mixture was stirred for 2 hours in a hydrogen atmosphere at room temperature. The reaction mixture was concentrated, and the residue was subjected to distribution with dichloromethan and saturated sodium bicarbonate. The aqueous layer was extracted with dichloromethane, and the extract was combined with the organic layer. The mixture was dried with MgSO₄, and the solvent was distilled off under reduced pressure. The residue was purified by silica gel column chromatography to give colorless powders (5.13 g, 60%). Thus obtained compound (100 mg) was dissolved in ethyl acetate (4 ml), and to the solution was added 1N solution of hydrogen chloride in ether (0.3 ml) under ice-cooling and the mixture was stirred for 10 minutes under ice-cooling. The reaction mixture was concentrated under reduced pressure, and the residue was crystallized from ethyl acetate-ether to give hydrochloride of the titled compound (95 mg) was obtained as white crystals.
m.p. 162-165°C.

| Elemental Analysis for C₃₅H₃₀N₄O₃SF₂·2.0HCl·1.0H₂O: | | | |
|---|---|---|---|
| | C(%) | H(%) | N(%) |
| Calcd. | 58.74 ; | 4.79 ; | 7.83 |
| Found | 58.44 ; | 4.72 ; | 7.66. |

IR (KBr): 1715, 1659, 1537, 1473 cm⁻¹.

### Example 10

Starting from the compounds which are produced in Example 8, compounds which are produced in accordance with the method described in Example 9 are set forth in Table 11.

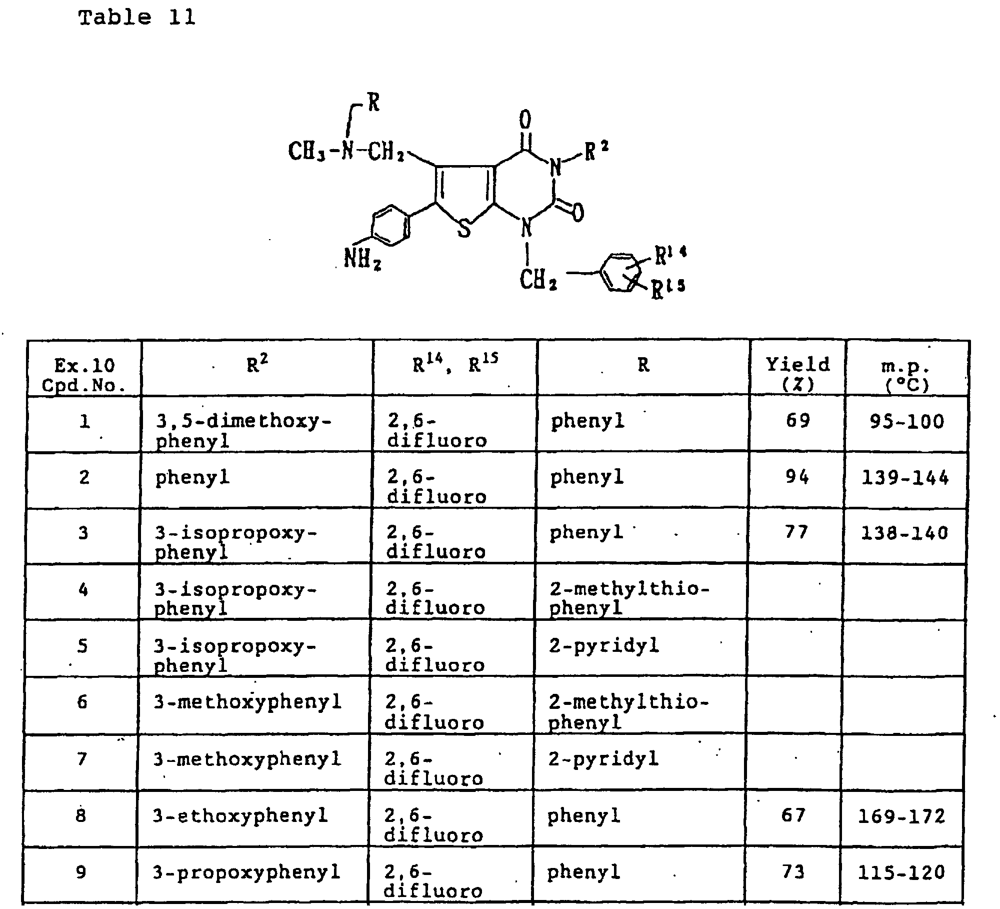

### Example 11

### Production of 2,4(1H,3H)-dioxo-1-(2,6-difluorobenzyl)-5-(N-benzyl-N-methylaminomethyl)-6-(4-formamidophenyl)-3-phenylthieno[2,3-d]pyrimidine:

Formic acid (0.5 ml, 13.3 mmol) was added to acetic anhydride (1.0 ml, 10.6 mmol) under ice-cooling, the mixture was stirred for one hour at 50°C to give formic acid-acetic acid anhydride. To a tetrahydrofuran (10 ml) solution of the compound 2 (200 mg, 0.34 mmol) obtained in Example 10 was added the formic acid-acetic acid anhydride (0.3 ml) under ice-cooling and the mixture was stirred for 30 minutes. The mixture was stirred for one hour. The reaction mixture was concentrated under reduced pressure. The residue was purified by silica gel column chromatography to give colorless solid substance (125 mg) of the titled compound.
m.p. 194-196°C.

| Elemental Analysis for C₃₅H₃₀N₄O₃SF₂·0.5H₂O: | | | |
|---|---|---|---|
| | C(%) | H(%.) | N(%) |
| Calcd. | 66.55 ; | 4.63 ; | 8.87 |
| Found | 66.74 ; | 4.56 ; | 8.88. |

¹H-NMR (300MHz, CDCl₃) δ: 3.57(2H,s), 3.90(2H,s), 5.37(2H,s), 6.90-7.30(12H,m), 7.34-7.79(6H,m), 8.42(1H,s).
IR (KBr): 1715, 1665, 1531, 1467 cm⁻¹.

### Example 12

Starting from the compounds which are produced in Example 9 or 10, compounds which are produced in accordance with the method described in Example 11 are set forth in Table 12.

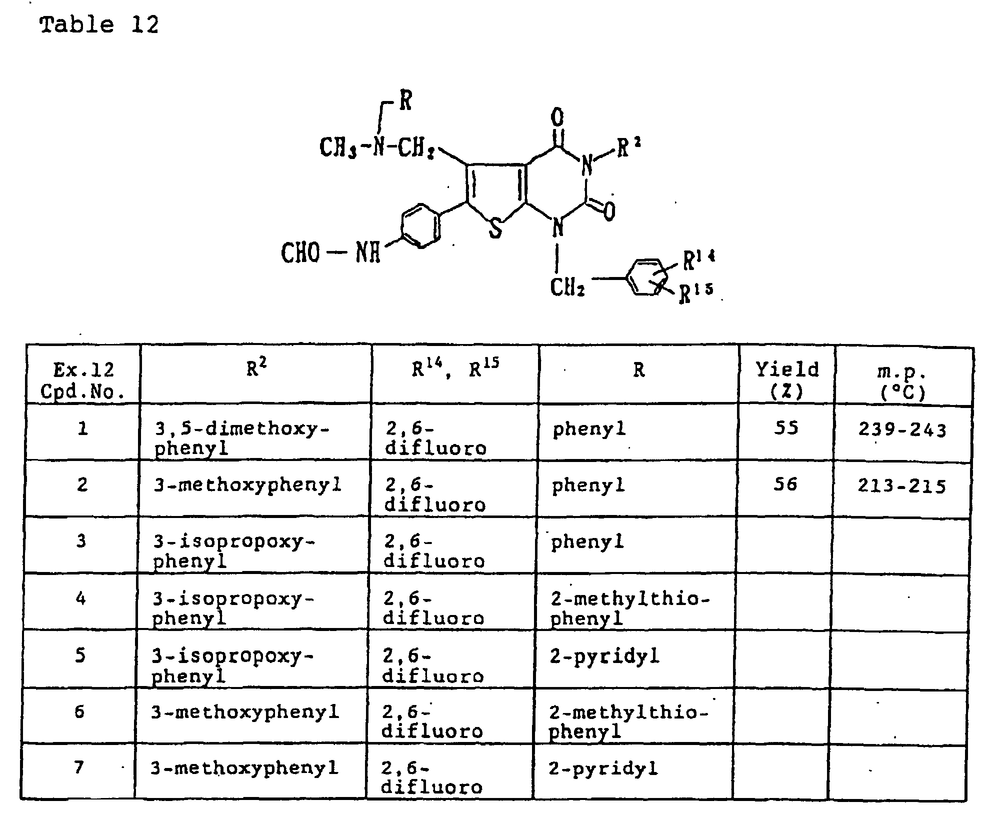

### Example 13

### Production of 2,4(1H,3H)-dioxo-1-(2,6-difluorobenzyl)-5-(N-benzyl-N-methylaminomethyl)-6-(4-methylaminophenyl)-3-(3-methoxyphenyl)thieno[2,3-d]pyrimidine hydrochloride:

To a tetrahydrofuran (30 ml) solution of the compound 2 (730 mg, 1.12 mmol) obtained in Example 12 was added dimethylsulfid borane (0.28 ml, 2.8 mmol) under ice-cooling, and the mixture was heated for 2 hours under reflux. After adding hydrochloric acid (pH<2) and then heating under reflux for 1 hour, the resultant was concentrated and the residue was subjected to distribution with dichloromethane and saturated sodium bicarbonate. The aqueous layer was extracted with dichloromethane, the extract was combined with organic layer, the mixture was dried with MgSO₄ and the solvent was distilled off under reduced pressure. The residue was purified by silica gel column chromatography to give colorless powder (610 mg, 85%). To the ethyl acetate (4 ml) solution of thus obtained compound was added 1N solution of hydrogen chloride in ether (0.3 ml under ice-cooling, and the mixture was stirred for 10 minutes under ice-cooling. The residue obtained by concentrating the reaction mixture under reduced pressure was subjected to crystallization to give white crystals (95 mg) of hydrochloride of the titled compound.
m.p. 155-160°C.

| Elemental Analysis for C₃₅H₃₀N₄O₃SF₂ ·2.0HCl · 0.5AcOEt · 3.0H₂O: | | | |
|---|---|---|---|
| | C(%) | H(%) | N(%) |
| Calcd. | 56.36 ; | 5.47 ; | 6.91 |
| Found | 56.08 ; | 5.22 ; | 6.86. |

IR (KBr): 1715, 1663, 1607, 1543, 1475 cm⁻¹.

### Example 14

### Production of 2,4(1H,3H)-dioxo-1-(2,6-difluorobenzyl)-6-(4-propionylaminophenyl)-5-(N-benzyl-N-methylaminomethyl)-3-(3-methoxyphenyl)thieno[2,3-d]pyrimidine hydrochloride:

To a dichloromethane (10 ml) solution of the compound (250 mg, 0.38 mmol) obtained in Example 9 were added triethylamine (0.053 mg, 0.38 mmol) and propionyl chloride (0.033 ml, 0.38 mmol) under ice-cooling, and the mixture was stirred one hour. The reaction mixture was subjected to distribution with dichloromethane and saturated sodium bicarbonate. The aqueous layer was extracted with dichloromethane, the extracts were combined, the combined extracts were washed with an aqueous solution of sodium chloride and dried with MgSO₄, and the solvent was distilled off under reduced pressure. The residue was purified by silica gel column chromatography to give colorless oily substance (220 mg, 82%). To an ethyl acetate (4 ml) solution of thus obtained acyl derivative was added 1N solution of hydrogen chloride in ether (0.3 ml) under ice-cooling and the mixture was stirred for 10 minutes under ice-cooling. The reaction mixture was concentrated under reduced pressure, the residue was crystallized to give white crystals of hydrochloride (213 mg) of the titled compound.
m.p. 218-224°C.
IR (KBr): 1713, 1665, 1601, 1543, 1475 cm^{-1.}

### Example 15

Starting from the compounds which are produced in Example 9 or 10, compounds which are produced in accordancd with the method described in Example 14 are set forth in Table 13.

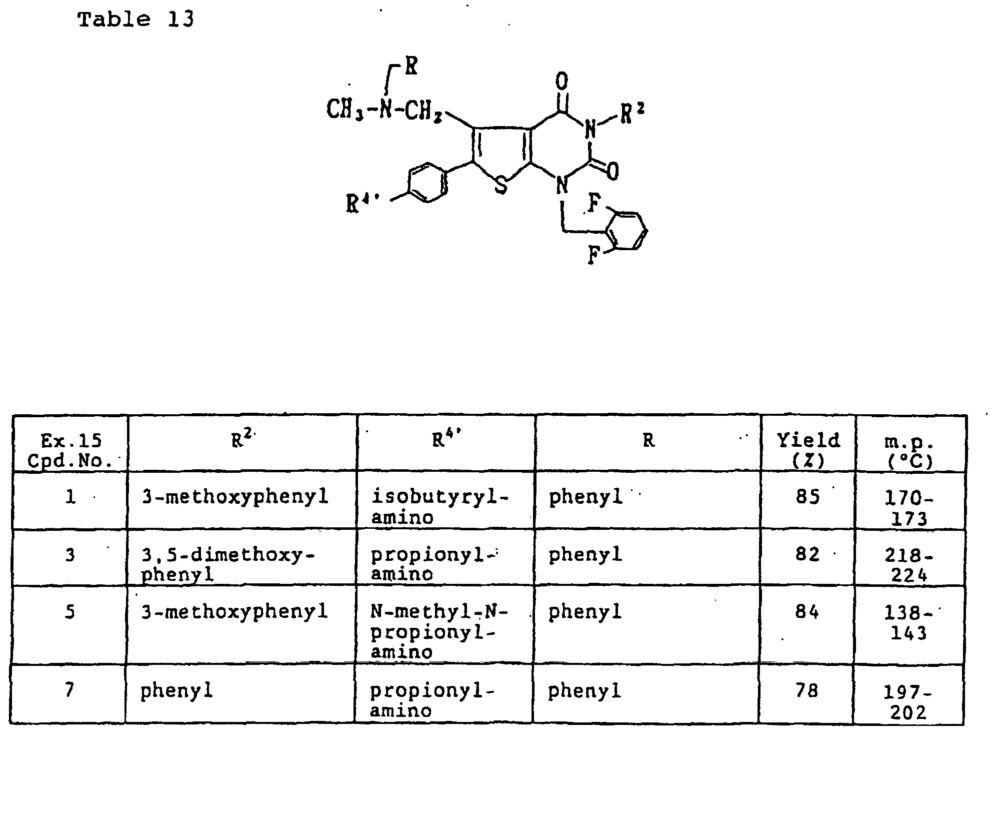

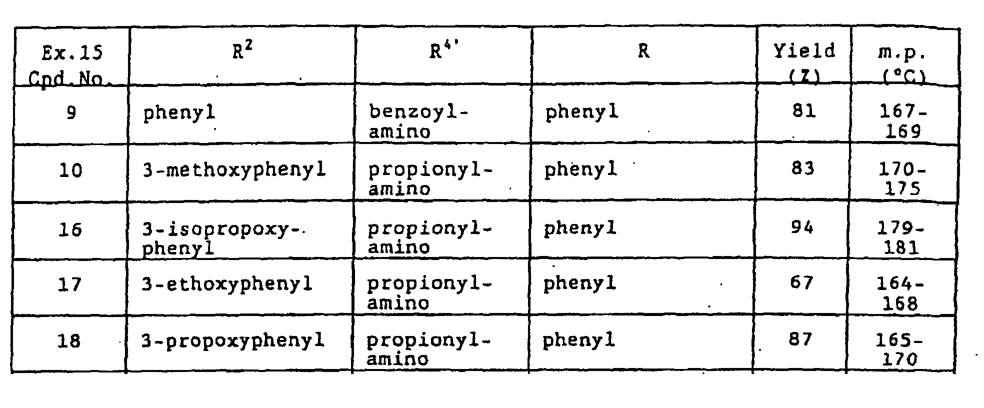

### Example 16

In substantially the same procedure as described in Example 14, using the compound which are obtained in Example 9 or 10 and anhydrous trifluoro acetic acid in place of propionyl chloride, trifluoroacetylamino derivative are obtained. To the derivative is added halogeno derivative (e.g. propyl bromide, isopropyl bromide) in the presence of an appropriate base (e.g. potassium carbonate) in a solvent (e.g. dimethylformamide) which does not affect the reaction, the mixture is stirred for 1 to 6 hours at room temperature. To the reaction mixture is added 2N aqueous sodium hydroxide solution for hydrolysis for 1 to 2 hours to give compounds set forth in Table 14.

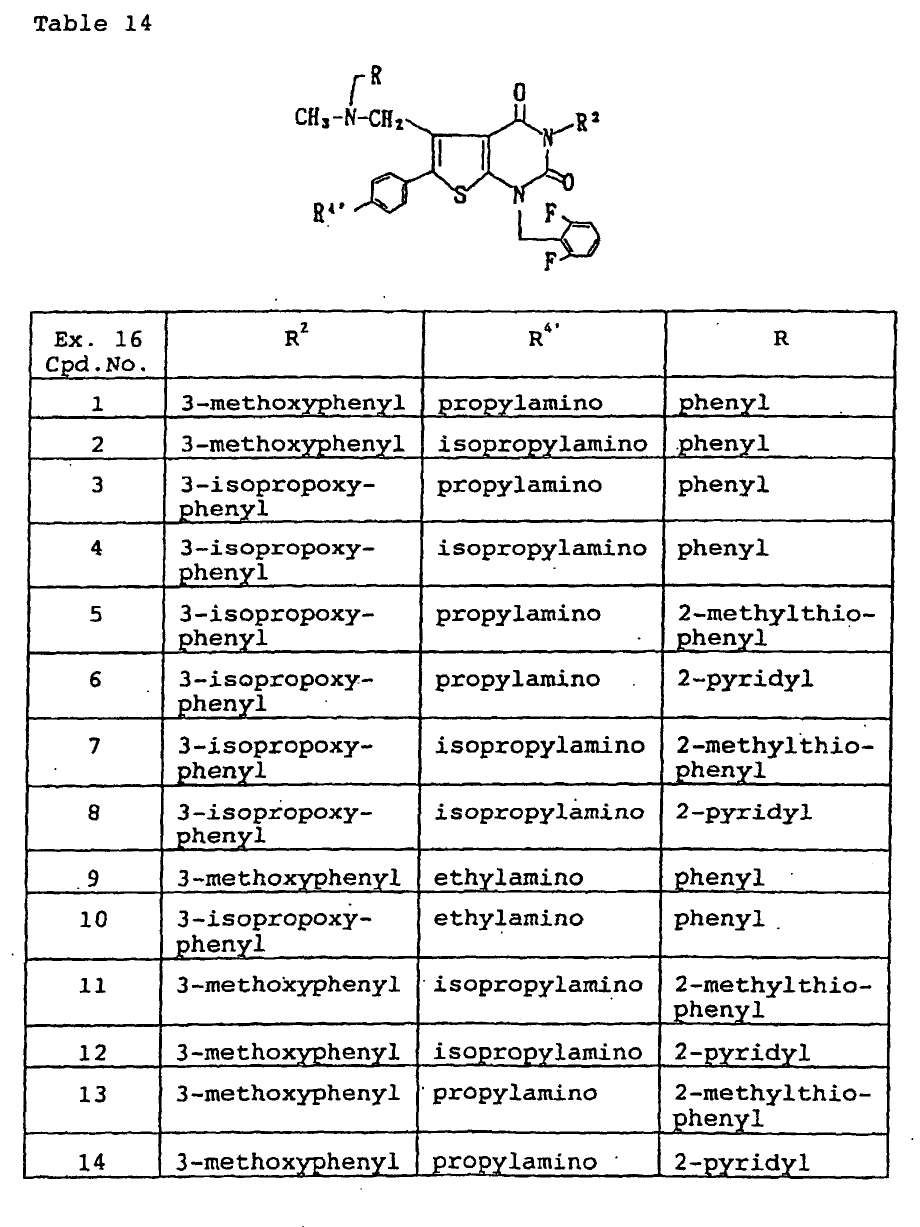

### Example 17

Employing the compounds which are obtained in Example 9 or 10 as starting compounds, the compounds set forth in Table 15 are produced by reacting the starting compounds with isoamyl nitrite, vinyl compound and palladium compound (e.g. tetrakistriphenylphosphine palladium, dibenzylideneacetone palladium) in acetic acid under stirring at a room temperature or under heating for 1 to 6 hours.

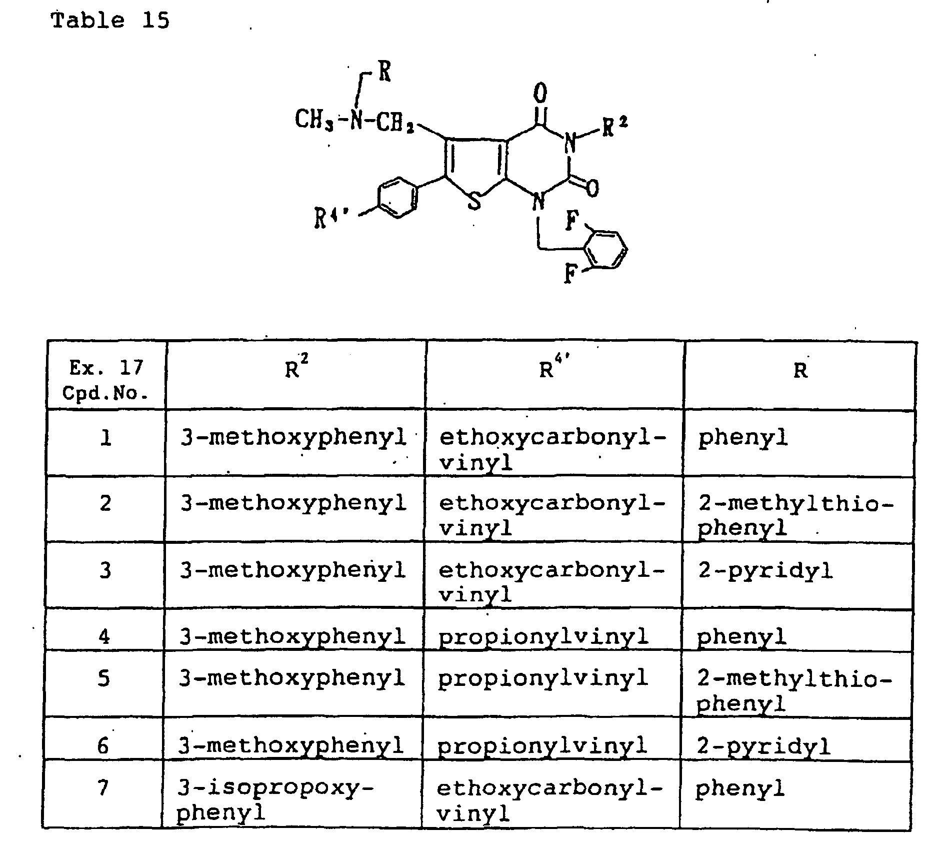

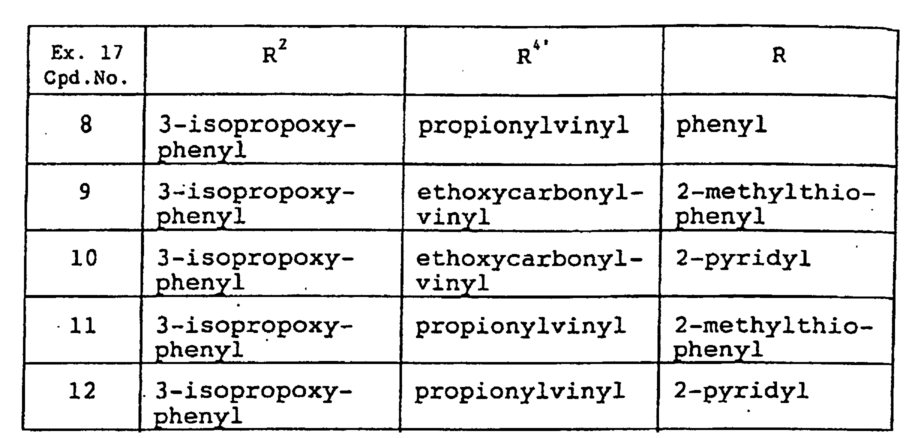

### Example 18

To a mixture of the compound 30 or 31 which are obtained in Reference Example 9, a small amount of arylborric acid derivative, 2M aqueous sodium carbonate solution and 1,2-dimethoxyethane, is added a catalytic amount of tetrakis(triphenylphosphine)palladium(0), and thus obtained mixture is stirred under reflux for 2 hours. To the resulting compound, N-methylbenzylamino group is introduced in accordance with the method described in Reference Example 26 and Example 1 to give compounds set forth in Table 16.

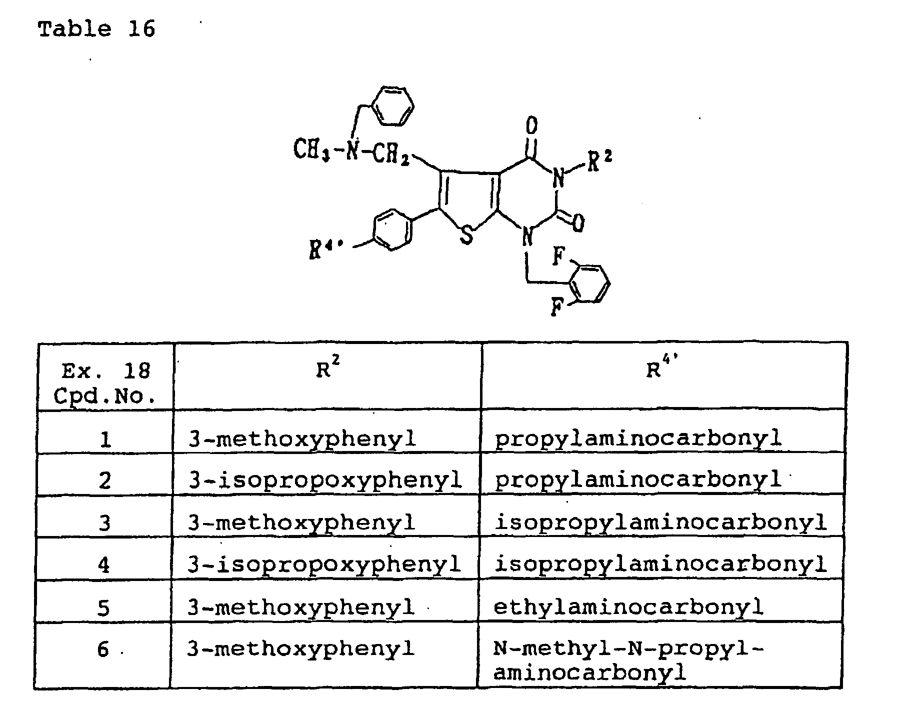

### Example 19

To the compounds which are obtained in Example 2, 3 equivalents of dimethylsulfide and 3 equivalents of aluminium chloride are added in dichloromethane under ice-cooling. The mixture is stirred for 1 to 4 hours to give R⁴ phenol derivative. Thus obtained compound, a small amount of an alkyl halide (e.g. chloro acetone) and a base (e.g. potassium carbonate) are mixed in dimethylformamide to produce compounds set forth in Table 17.

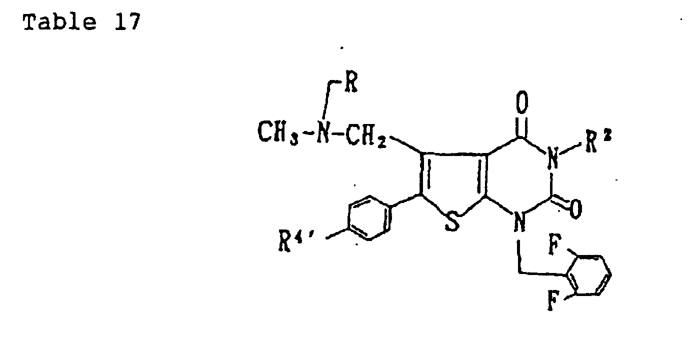

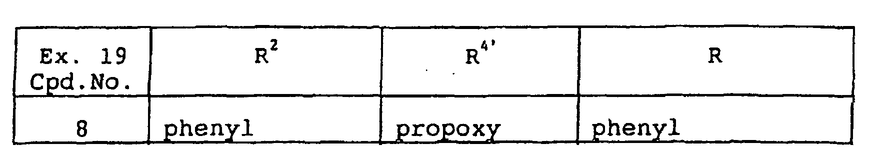

### Example 20

Using the compound produced in Example 4 (100 mg), lactose (165 mg), corn starch (5 mg), polyvinyl alcohol (4 mg) and magnesium stearate (1 mg), a tablet is prepared by a conventional method.

### Example 21

The compound produced in Example 4 (5 g) is dissolved in distilled water for injection to make the whole volume 100 ml. The solution is subjected to sterilized filtration with 0.22 µm membrane filter (manufactured by Sumitomo Electric Industries, Ltd. or by Zartolius, Inc.), 2 ml each of which is distributed to sterilized vials, followed by lyophilization by a conventional means to give lyophilized injectable solution of 100 mg/vial.

### Example 22

Using the compound 1 produced in Example 15 (100 mg), lactose (165 mg), corn starch (25 mg), polyvinyl alcohol (4 mg) and magnesium stearate (1 mg), a tablet is prepared by a conventional method.

### Example 23

The compound 1 produced in Example 15 (5 g) is dissolved in distilled water for injection to make the whole volume 100 ml. This solution is subjected to . sterilized filtration with 0.22 µm membrane filter (manufactured by Sumitomo Electric Industries, Ltd. or Zartolius, Inc.), 2 ml each of which is distributed to sterilized vials, followed by lyophilization by a conventional means to prepare lyophilized injectable solution of 100 mg/vial.

### Example 24

| | | |
|---|---|---|
| (1) | Compound produced in Example 4 or the compound 1 of Example 15 | 5 g |
| (2) | Lactose.crystalline cellulose (granules) | 330 g |
| (3) | D-mannitol | 29 g |
| (4) | Low-substituted hydroxypropyl cellulose | 20 g |
| (5) | Talc | 25 g |
| (6) | Hydroxypropyl cellulose | 50 g |
| (7) | Aspartame | 3 g |
| (8) | Dipotassium glycyrrhetinate | 3 g |
| (9) | Hydroxypropylmethyl cellulose 2910 | 30 g |
| (10) | Titanium oxide | 3.5 g |
| (11) | Yellow iron sesquioxide | 0.5 g |
| (12) | Light silicic acid anhydride | 1 g |

In refined water are suspended or dissolved (1), (3), (4), (5), (7) and (8). The nuclear granule of (2) is coated with the suspension or solution to prepare raw fine granules, which are coated with (9)-(11) to prepare coated fine granules, which are mixed with (12), to give 500 g of fine granules containing 1% of the compound produced in Example 4 or the compound 1 of Example 15. 500 mg each of thus-prepared fine granules is packed.

### Industrial Applicability

A thienopyrimidine derivative (I) of the present invention is effective as a propylactic or therapeutic agent for the prevention or treatment of several hormone dependent diseases, for example, a sex hormone dependent cancer (e.g. prostatic cancer, cancer of uterine cervix, breast cancer, pituitary adenoma), benign prostatic hypertrophy, myoma of the uterus, endometriosis, precocious puberty, amenorrhea, premenstrual syndrome, polycystic ovary syndrome and acne vulgaris; is effective as a fertility controlling agent in both sexes (e.g. a pregnancy controlling agent and a menstrual cycle controlling agent); can be used as a contraceptive of male or female, as an ovulation-inducing agent of female; can be used as an infertility treating agent by using a rebound effect owing to a stoppage of administration thereof; is useful as modulating estrous cycles in animals in the field of animal husbandry, as an agent for improving the quality of edible meat or promoting the growth of animals; and is useful as an agent of spawning promotion in fish.

## Claims

1. A compound of the formula 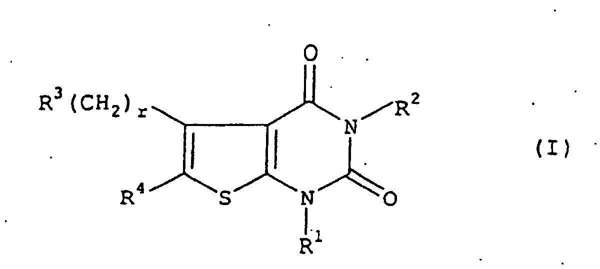 wherein
R¹ is C₆₋₁₄ aryl-methyl group which is substituted by 1 to 3 of
(i) halogen,
(ii) C₁₋₆ alkylthio and
(iii) C₁₋₆ alkoxy;
R² is
(1) C₁₋₆ alkyl group which is unsubstituted or substituted by C₁₋₃ alkoxy,
(2) C₆₋₁₄ aryl which is unsubstituted or substituted by 1 to 3 of (i) hydroxyl, (ii) C₁₋₆ alkoxy, (iii) a group of the formula: -S(O)ₙ-R⁶ in which n is an integer of 0 to 2 and R⁶ is C₁₋₃ alkyl and (iv) halogen,
(3) C₇₋₁₀ aralkyl or
(4) C₃₋₁₀ cycloalkyl;
R³ is a group of the formula: 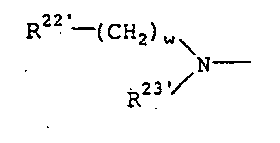 wherein
R^{22'} is a phenyl or pyridyl, these groups being unsubstituted or substituted by a group of the formula: -S(O)ₙ-R⁶ in which n is an integer of 0 to 2 and R⁶ is C₁₋₃ alkyl,
w is an integer of 0 to 3, and
R^{23'} is a hydrogen or C₁₋₆ alkyl;
R⁴ is phenyl substituted by
(1) amino,
(2) C₁₋₈ acyl,
(3) N-substituted C₁₋₇ alkylcarbamoyl,
(4) nitro,
(5) C₁₋₃ alkoxy which is unsubstituted or substituted by C₁₋₃ alkoxy,
(6) a group of the formula: 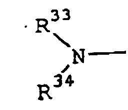 wherein
R³³ is (i) C₁₋₆ alkyl, (ii) C₁₋₃ acyl which is unsubstituted or substituted by C₁₋₃ alkoxy, (iii) C₁₋₃ alkoxy which is unsubstituted or substituted by C₁₋₄ acyl, (iv) benzoyl or (v) formyl, and
R³⁴ is (i) hydrogen or (ii) C₁₋₆ alkyl, or
(7) C₂₋₄ alkenyl which is unsubstituted or substituted by C₁₋₃ alkoxy-carbonyl or C₁₋₃ alkyl-carbonyl; and
r is 1;
or 2,4(1H,3H)-dioxo-1-(2,6-difluorobenzyl)-6-(4-isobutyrylaminophenyl)-5-(N-benzyl-N-methylaminomethyl)-3-(3-methoxyphenyl)thieno[2,3-d]pyrimidine;
or a salt thereof.

2. The compound of claim 1, which is 2,4(1H,3H)-dioxo-6-(4-methoxyphenyl)-3-phenyl-1-(2-chloro-6-fluorobenzyl)-5-(N-benzyl-N-methylaminomethyl)thieno[2,3-d]pyrimidine or a salt thereof.

3. The compound of claim 1, which is 2,4(1H,3H)-dioxo-1-(2,6-difluorobenzyl)-6-(4-propionylaminophenyl)-5-(N-benzyl-N-methylaminomethyl)-3-(3-methoxyphenyl)thieno[2,3-d]pyrimidine or a salt thereof.

4. A method for producing a compound of the formula: 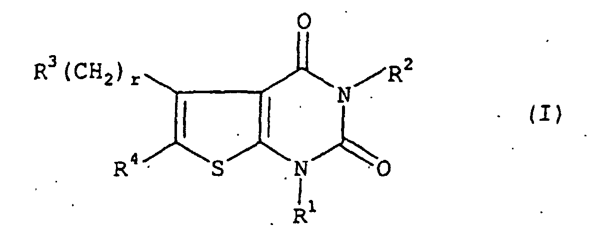 wherein R¹, R², R³, R⁴ and r are as defined in claim 1 or a salt thereof, which comprises reacting a compound of the formula: 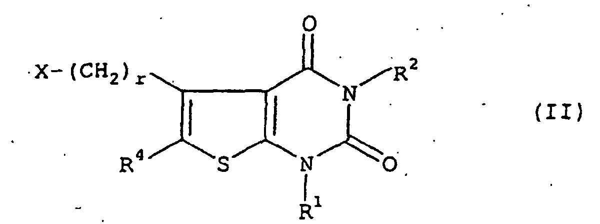 wherein R¹, R², R⁴ and r have the same meaning as defined above and X is a leaving group, or a salt thereof, with a compound of the formula:
R³-H
wherein R³ has the same meaning as defined above, or a salt thereof.

5. A pharmaceutical composition, which comprises a compound as defined in claim 1 and a carrier, excipient or diluent therefor.

6. The composition of claim 5, which is a gonadotropin-releasing hormone antagonistic composition.

7. The composition of claim 5, which is for preventing or treating a sex hormone dependent disease.

8. The compound of claim 1, which is for medicinal use.

9. Use of a compound as defined in claim 1 for producing a gonadotropin-releasing hormone antagonistic composition for antagonizing gonadotropin-releasing hormone in a mammal suffering from a gonadotropin-releasing hormone derived disorder.

10. The use according to claim 9, wherein the gonadotropin-releasing hormone derived disorder is a sex hormone dependent disease.

## Patentansprüche

1. Verbindung der Formel 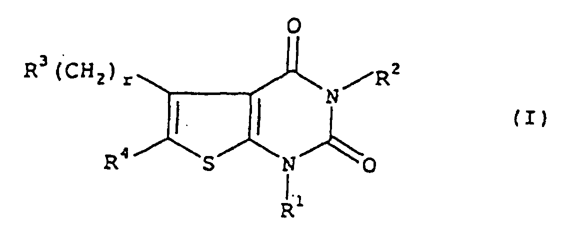 wobei
R¹ eine C₆₋₁₄-Arylmethylgruppe ist, die mit 1 bis 3 der folgenden substituiert ist:
(i) Halogen
(ii) C₁₋₆-Alkylthio; und
(iii) C₁₋₆-Alkoxy;
R² folgendes ist:
(1) eine C₁₋₆-Alkylgruppe, die unsubstituiert oder mit C₁₋₃-Alkoxy substituiert ist;
(2) C₆₋₁₄-Aryl, das unsubstituiert oder mit 1 bis 3 der folgenden substituiert ist: (i) Hydroxy, (ii) C₁₋₆-Alkoxy, (iii) einer Gruppe der Formel -S(O)ₙ-R⁶, wobei n eine ganze Zahl von 0 bis 2 ist und R⁶ C₁₋₃-Alkyl ist, und (iv) Halogen;
(3) C₇₋₁₀-Aralkyl; oder
(4) C₃₋₁₀-Cycloalkyl;
R³ eine Gruppe der Formel 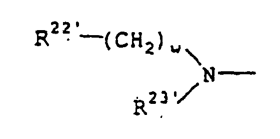 ist, wobei
R^{22'} Phenyl oder Pyridyl ist, wobei diese Gruppen unsubstituiert oder mit einer Gruppe der Formel -S(O)ₙ-R⁶, wobei n eine ganze Zahl von 0 bis 2 ist und R⁶ C₁₋₃-Alkyl ist, substituiert sind;
w eine ganze Zahl von 0 bis 3 ist; und
R^{23'} Wasserstoff oder C₁₋₆-Alkyl ist;
R⁴ Phenyl ist, das substituiert ist mit:
(1) Amino;
(2) C₁₋₈-Acyl;
(3) N-substituiertem C₁₋₇-Alkylcarbamoyl;
(4) Nitro;
(5) C₁₋₃-Alkoxy, das unsubstituiert oder mit C₁₋₃-Alkoxy substituiert ist;
(6) einer Gruppe der Formel: 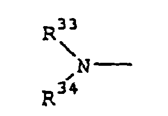 wobei
R³³ folgendes ist: (i) C₁₋₆-Alkyl, (ii) C₁₋₃-Acyl, das unsubstituiert oder mit C₁₋₃-Alkoxy substituiert ist, (iii) C₁₋₃-Alkoxy, das unsubstituiert oder mit C₁₋₄-Acyl substituiert ist, (iv) Benzoyl oder (v) Formyl; und
R³⁴ (i) Wasserstoff oder (ii) C₁₋₆-Alkyl ist; oder
(7) C₂₋₄-Alkenyl, das unsubstituiert oder mit C₁₋₃-Alkoxycarbonyl oder C₁₋₃-Alkylcarbonyl substituiert ist; und
r = 1 ist;
oder 2,4(1H,3H)-Dioxo-1-(2,6-difluorbenzyl)-6-(4-isobutyrylaminophenyl)-5-(N-benzyl-N-methylaminomethyl)-3-(3-methoxyphenyl)thieno[2,3-d]pyrimidin;
oder ein Salz davon.

2. Verbindung gemäß Anspruch 1, bei der es sich um 2,4(1H,3H)-Dioxo-6-(4-methoxyphenyl)-3-phenyl-1-(2-chlor-6-fluorbenzyl)-5-(N-benzyl-N-methylaminomethyl)thieno[2,3-d]pyrimidin oder ein Salz davon handelt.

3. Verbindung gemäß Anspruch 1, bei der es sich um 2,4(1H,3H)-Dioxo-1-(2,6-difluorbenzyl)-6-(4-propionylaminophenyl)-5-(N-benzyl-N-methylaminomethyl)-3-(3-methoxyphenyl)thieno[2,3-d]pyrimidin oder ein Salz davon handelt.

4. Verfahren zur Herstellung einer Verbindung der Formel: 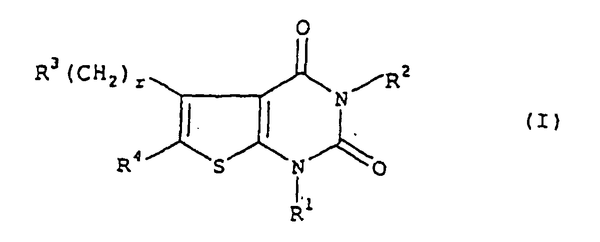 wobei R¹, R², R³, R⁴ und r wie in Anspruch 1 definiert sind, oder eines Salzes davon, umfassend das Umsetzen einer Verbindung der Formel: 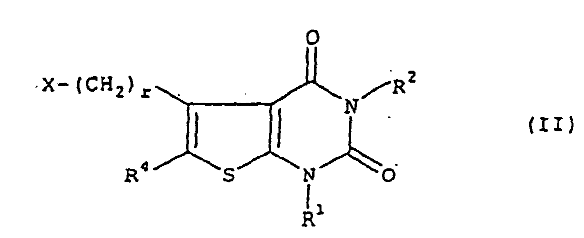 wobei R¹, R², R⁴ und r dasselbe wie oben bedeuten und X eine Abgangsgruppe ist, oder eines Salzes davon mit einer Verbindung der Formel:
R³-H,
wobei R³ dasselbe wie oben bedeutet, oder einem Salz davon.

5. Pharmazeutische Zusammensetzung, die eine Verbindung gemäß Anspruch 1 und einen Träger, Arzneimittelhilfsstoff oder ein Verdünnungsmittel dafür umfasst.

6. Zusammensetzung gemäß Anspruch 5, bei der es sich um eine gegenüber gonadotropinfreisetzendem Hormon antagonistische Zusammensetzung handelt.

7. Zusammensetzung gemäß Anspruch 5, die zur Prävention oder Behandlung einer sexualhormonabhängigen Krankheit dient.

8. Verbindung gemäß Anspruch 1, die zur medizinischen Verwendung dient.

9. Verwendung einer Verbindung, wie sie in Anspruch 1 definiert ist, zur Herstellung einer gegenüber gonadotropinfreisetzendem Hormon antagonistischen Zusammensetzung zum Antagonisieren von gonadotropinfreisetzendem Hormon bei einem Säuger, der unter einer auf das gonadotropinfreisetzende Hormon zurückgehenden Störung leidet.

10. Verwendung gemäß Anspruch 9, wobei die auf das gonadotropinfreisetzende Hormon zurückgehende Störung eine sexualhormonabhängige Krankheit ist.

## Revendications

1. Composé de formule 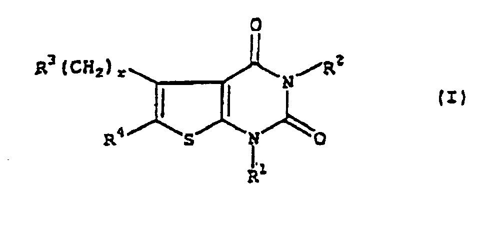 dans laquelle
R¹ est un groupe C₆₋₁₄-arylméthyle qui est substitué par de 1 à 3 groupes parmi
(i) un halogène,
(ii) un C₁₋₆-alkylthio et
(iii) un alcoxy en C₁₋₆ ;
R² est
(1) un groupe alkyle en C₁₋₆ qui est non substitué ou substitué par un alcoxy en C₁₋₃,
(2) un aryle en C₆₋₁₄ qui est non substitué ou substitué par de 1 à 3 groupes parmi (i) un hydroxy, (ii) un alcoxy en C₁₋₆, (iii) un groupe de formule : -S(O)ₙ-R⁶ dans laquelle n est un entier de 0 à 2 et R⁶ est un alkyle en C₁₋₃, et (iv) un halogène,
(3) un aralkyle en C₇₋₁₀ ou
(4) un cycloalkyle en C₃₋₁₀ ;
R³ est un groupe de formule : 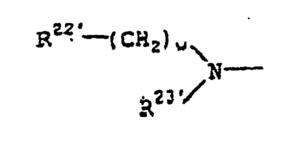 dans laquelle
R^{22'} est un phényle ou un pyridyle, ces groupes étant non substitués ou substitués par un groupe de formule : -S(O)ₙ-R⁶ dans laquelle n est un entier de 0 à 2 et R⁶ est un alkyle en C₁₋₃,
w est un entier de 0 à 3, et
R^{23'} est un hydrogène ou un alkyle en C₁₋₆ ;
R⁴ est un phényle substitué par
(1) un amino,
(2) un acyle en C₁₋₈,
(3) un C₁₋₇-alkylcarbamoyle N-substitué,
(4) un nitro,
(5) un alcoxy en C₁₋₃ qui est non substitué ou substitué par un alcoxy en C₁₋₃,
(6) un groupe de formule : 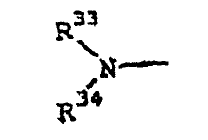 dans laquelle
R³³ est (i) un alkyle en C₁₋₆, (ii) un acyle en C₁₋₃ qui est non substitué ou substitué par un alcoxy en C₁₋₃, (iii) un alcoxy en C₁₋₃ qui est non substitué ou substitué par un acyle en C₁₋₄, (iv) un benzoyle ou (v) un formyle, et
R³⁴ est (i) un hydrogène ou (ii) un alkyle en C₁₋₆, ou
(7) un alcényle en C₂₋₄ qui est non substitué ou substitué par un C₁₋₃-alcoxycarbonyle ou un C₁₋₃-alkylcarbonyle; et
r vaut 1 ;
ou la 2,4(1H,3H)-dioxo-1-(2,6-difluorobenzyl)-6-(4-isobutyrylaminophényl)-5-(N-benzyl-N-méthylaminométhyl)-3-(3-méthoxyphényl)thiéno[2,3-d]pyrimidine ;
ou l'un de ses sels.

2. Composé selon la revendication 1, qui est la 2,4-(1H,3H)-dioxo-6-(4-méthoxyphényl)-3-phényl-1-(2-chloro-6-fluorobenzyl)-5-(N-benzyl-N-méthylaminométhyl)thiéno[2,3-d]pyrimidine ou l'un de ses sels.

3. Composé selon la revendication 1, qui est la 2,4-(1H,3H)-dioxo-1-(2,6-difluorobenzyl)-6-(4-propionylaminophényl)-5-(N-benzyl-N-méthylaminométhyl)-3-(3-méthoxyphényl)thiéno[2,3-d]pyrimidine ou l'un de ses sels.

4. Procédé de production d'un composé de formule : 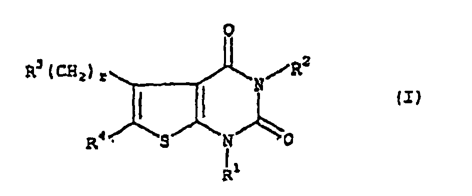 dans laquelle R¹, R², R³, R⁴ et r sont tels que définis dans la revendication 1, ou de l'un de ses sels, comprenant la réaction d'un composé de formule : 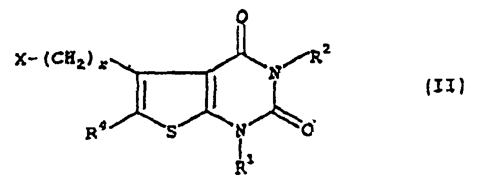 dans laquelle R¹, R², R⁴ et r présentent la même signification que celle définie ci-dessus et X est un groupe partant, ou de l'un de ses sels, avec un composé de formule :
R³-H
dans laquelle R³ présente la même signification que celle définie ci-dessus, ou l'un de ses sels.

5. Composition pharmaceutique comprenant un composé tel que défini dans la revendication 1 et un support, un excipient ou un diluant pour celui-ci.

6. Composition selon la revendication 5, qui est une composition antagoniste de la gonadolibérine.

7. Composition selon la revendication 5, qui est destinée à empêcher ou à traiter une maladie dépendante des hormones sexuelles.

8. Composé selon la revendication 1, qui est destiné à un usage médicinal.

9. Utilisation d'un composé tel que défini dans la revendication 1 pour la production d'une composition antagoniste de la gonadolibérine pour antagoniser la gonadolibérine chez un mammifère souffrant d'un trouble issu de la gonadolibérine.

10. Utilisation selon la revendication 9, dans laquelle le trouble issu de la gonadolibérine est une maladie dépendante des hormones sexuelles.
